# EUROPEAN PATENT APPLICATION

(11) **EP 3 653 722 A1**
(43) Date of publication of application: **20.05.2020**
(21) Application number: 18832456.0
(22) Date of filing: 12.04.2018
(51) Int. Cl.: C12Q 1/68

(54) **APPLICATION OF CAS PROTEIN, METHOD FOR DETECTING TARGET NUCLEIC ACID MOLECULE AND KIT**

(30) Priority: 14.07.2017 CN 201710573752
(71) Applicant: Shanghai Tolo Biotechnology Company Limited, Shanghai 200032 (CN)
(72) Inventor: WANG, Jin, Shanghai 200031 (CN); CHENG, Qiuxiang, Shanghai 201899 (CN); LI, Shiyuan, Shanghai 200031 (CN); LI, Xiaoyan, Shanghai 201899 (CN); LI, Linxian, Shanghai 200031 (CN)
(74) Representative: Cooley (UK) LLP
(86) International application number: PCT/CN2018/082769
(87) International publication number: WO 2019/011022

(57) **Abstract**

Provided in the present invention are use of a Cas protein, a method for detecting a target nucleic acid molecule, and a kit, the method for detecting a target nucleic acid molecule comprises adding a guide RNA, Cas12a and a nucleic acid probe into a reaction system comprising a target nucleic acid molecule to be detected, and carrying out detection after the reaction is complete.

## Description

### TECHNICAL FIELD

The present invention belongs to the field of biotechnology, and in particular to a method for detecting a target nucleic acid molecule.

### BACKGROUND

A specific nucleic acid detection method has an important application value, such as pathogen detection, genetic disease detection, etc. In an aspect of pathogen detection, since each pathogen microorganism has its unique characteristic nucleic acid molecular sequence, it is possible to develop nucleic acid molecular detection for specific species, also known as nucleic acid diagnostic (NAD), which is of great significance in the fields of food safety, environmental microorganism pollution detection, human pathogen infection, etc. Another aspect is the detection of single nucleotide polymorphism (SNP) in human or other species. Understanding the relationship between a genetic variation and a biological function at the genomic level provides a new perspective for modern molecular biology. SNP is closely related to biological functions, evolution and diseases, so the development of SNP detection and analysis technologies is particularly important.

At present, many NAD methods have been established, mainly for the detection of a specific DNA molecule, and there are also some methods for RNA molecules. Generally speaking, a DNA molecule is very stable, so a test sample can come from a series of complex biological samples; while RNA is very easy to degrade, so it needs to be handled with great care. In the 1970s, a detection method using restriction endonuclease digestion was established. Later, methods such as Southern, Northern and dot blot hybridization were developed for specific detection of a nucleic acid molecule. In 1985, when PCR became a conventional experimental method, it led to an exponential improvement in molecular biology. Currently established specific nucleic acid molecule detection usually needs to be performed in two steps, the first step being the amplification of a target nucleic acid and the second step being the detection of the target nucleic acid. PCR technology is an amplification method that is first established and most commonly used at present. Currently, based on the PCR method, a fluorescent-labeled probe is introduced, such that the amplification situation of a target can be detected in real time, which is called Realtime PCR. The Realtime PCR is not only a fast and highly-sensitive detection method, but also a method for quantitative analysis. In addition to the PCR amplification method, many alternative methods have been established, such as ligase chain reaction, branched DNA amplification, NASBA, SDA, transcription-mediated amplification, Loop-mediated isothermal amplification (LAMP), rolling circle amplification (RCA), Recombinase Polymerase Amplification (RPA), etc. The advantage of many of these alternative methods is isothermality. That is to say, only one temperature is needed to complete the reaction, without the need for a thermal cycling instrument like that used in PCR. Among nucleic acid detection methods, besides the Realtime PCR that can directly complete amplification and detection, the FISH (Fluorescence in situ hybridization) technology is the most commonly-used detection method - a method in which a labeled molecular probe is *in situ* hybridized with a complementary target sequence. In addition, detection methods such as next-generation sequencing technologies and Oxford Nanopore sequencing technologies have also been developed, but these methods usually require expensive experimental equipment.

The detection of SNPs also first requires amplification by a method such as PCR and the like, so as to obtain sufficient SNP site-containing region fragments for further detection. Commonly used methods include: primer extension, hybridization, ligation, and enzymatic cleavage. When the above methods are completed, a specific method needs to be utilized for detection, such as mass spectrometry detection, fluorescence detection, chemiluminescence detection, etc.

Although many detection methods have been developed for nucleic acid detection as described above, in certain cases, how to detect more quickly, simply and less expensively is still an important development direction, such as rapid detection of pathogenic bacteria in the field, rapid detection of drug-sensitive SNPs, etc. In 2016, Collins *et al.* developed a rapid and inexpensive method for detecting the Zika virus based on the characteristic of CRISPR-Cas9 of specifically recognizing and cleaving a target sequence. In 2017, Feng Zhang *et al.* established a rapid nucleic acid detection method utilizing a feature of "collateral effect" of CRISPR-Cas13a. The "collateral effect" means that Cas13a binds to a specific target RNA and then randomly cleaves other non-target RNAs (here RNA molecules are designed as an RNA fluorescence reporting system); rapid target RNA detection is realized by combining with an isothermal amplification technology RPA; and the team of Feng Zhang called this detection method as SHERLOCK (Specific High Sensitivity Enzymatic Reporter UnLOCKing). The SHERLOCK method involves the binding to an RNA template, so if DNA detection is required, the DNA needs to be firstly transcribed into an RNA template for detection; and given the instability of RNA, this method will undoubtedly increase the difficulty of operation.

In 2015, Feng Zhang *et al.* discovered a new CRISPR-related endoproteinase Cas12a (formerly known as Cpf1), which, like a commonly used Cas9 protein, is an RNA-guided specific DNA endonuclease; however, compared with Cas9, Casl2a has its own characteristics, for example, only a crRNA is needed to guide the specific cleavage of a double-stranded DNA and a sticky end is produced.

### SUMMARY

An objective of the present invention is to provide a method for detecting a target nucleic acid molecule.

Another objective of the present invention is to provide use of a Cas protein in a method for detecting a target nucleic acid molecule.

In a first aspect of the present invention, provided is a kit including a guide RNA, a Cas protein, a nucleic acid probe, and a buffer solution.

A method for detecting a target nucleic acid molecule includes adding a guide RNA, a Cas protein, a nucleic acid probe and a buffer solution into a reaction system containing a target nucleic acid molecule to be detected, and then detecting the target nucleic acid (especially by a method of detecting fluorescence intensity).

Preferably, the Cas protein is Cas12a or Cas protein having a collateral single-stranded DNA cleavage activity similar to that of the Cas12a.

Preferably, the Cas protein is Cas12a.

The Casl2a is preferably one of FnCas12a, AsCas12a, LbCas12a, Lb5Cas12a, HkCas12a, OsCas12a, TsCas12a, BbCas12a, BoCas12a, or Lb4Cas12a.

Preferably, the Cas12a is LbCas12a.

Preferably, the guide RNA refers to an RNA that guides the Cas protein to specifically bind to a target DNA.

In another preferred embodiment, the nucleic acid probe is a single-stranded DNA; the single-stranded DNA is preferably a fluorescently-labeled single-stranded DNA; the single-stranded DNA is preferably a fluorescent probe that is labeled with a fluorescent group HEX at a 5' terminal and labeled with a quenching group BHQ1 at a 3' terminal.

In another preferred embodiment, the method for detecting the nucleic acid probe is preferably a fluorescence detection method; and the fluorescence detection method is preferably a detection method using a microplate reader or a fluorescence spectrophotometer.

Preferably, the target nucleic acid molecule to be detected in the reaction system of the target nucleic acid molecule to be detected is obtained after amplification.

Preferably, the detection method of the present invention can be used for detecting a pathogenic microorganism, gene mutation, or a specific target DNA.

In another preferred embodiment, the Cas protein includes Cas12b (C2c1).

In a second aspect of the present invention, provided is use of a Cas protein in a method for detecting a target nucleic acid molecule, or in preparation of a formulation for detecting a target nucleic acid molecule.

In another preferred embodiment, when a target DNA, a guide RNA and a Cas protein form a ternary complex, the complex will cleave other single-stranded DNA molecules in the system.

Preferably, the guide RNA refers to an RNA that guides the Cas protein to specifically bind to a target DNA.

In a third aspect of the present invention, provided is a kit including a guide RNA, a Cas protein, and a nucleic acid probe.

In another preferred embodiment, the kit further includes a buffer solution.

In a fourth aspect of the present invention, provided is a detection system for detecting a target nucleic acid molecule, the system including:
(a) a Cas protein, which is Cas12a or a Cas protein having a collateral single-stranded DNA cleavage activity similar to that of the Cas12a;
(b) a guide RNA that guides the Cas protein to specifically bind to the target nucleic acid molecule; and
(c) a nucleic acid probe which is a single-stranded DNA;
wherein the target nucleic acid molecule is a target DNA.

In another preferred embodiment, the detection system further includes (d) a buffer solution.

In another preferred embodiment, the detection system further includes a target nucleic acid molecule to be detected.

In another preferred embodiment, the concentration of the target nucleic acid molecule to be detected in the detection system is 1-100 copies/microliter or 10¹⁵ copies/microliter, preferably 1-10 copies/microliter, and more preferably 1-5 copies/microliter.

In another preferred embodiment, in the detection system, the molar ratio of the nucleic acid probe to the target nucleic acid molecule is 10³: 1 to 10¹⁴: 1, and preferably 10⁴: 1 to 10⁷: 1.

In another preferred embodiment, the detection site of the target nucleic acid molecule is located at positions 1-12 downstream of the PAM sequence of the guide RNA.

In another preferred embodiment, the length of the guide RNA is 15-30 nt, and preferably 15-18 nt.

In another preferred embodiment, the target DNA includes a cDNA.

In another preferred embodiment, the target DNA is selected from a group consisting of a single-stranded DNA, a double-stranded DNA, or a combination thereof.

In another preferred embodiment, the nucleic acid probe carries a fluorescent group and a quenching group.

In another preferred embodiment, the fluorescent group and the quenching group are each independently located at the 5' terminal, 3' terminal and the middle portion of the nucleic acid probe.

In another preferred embodiment, the length of the nucleic acid probe is 3-300 nt, preferably 5-100 nt, more preferably 6-50 nt, and most preferably 8-20 nt.

In another preferred embodiment, the target nucleic acid molecule includes a target nucleic acid molecule derived from a group consisting of plants, animals, insects, microorganisms, viruses, or a combination thereof.

In another preferred embodiment, the target DNA is an artificially-synthesized or naturally occurring DNA.

In another preferred embodiment, the target DNA includes a wild-type or mutant DNA.

In another preferred embodiment, the target DNA includes a DNA obtained by reverse transcription of RNA or amplification, such as a cDNA, etc.

In another preferred embodiment, the Cas12a is selected from a group consisting of FnCas12a, AsCas12a, LbCas12a, Lb5Cas12a, HkCas12a, OsCas12a, TsCas12a, BbCas12a, BoCas12a, Lb4Cas12a, or a combination thereof; and more preferably, the Cas12a is LbCas12a.

In another preferred embodiment, the Cas protein having a collateral single strand DNA cleavage activity similar to that of Cas12a is selected from a group consisting of Cas12b (i.e., C2c1).

In another preferred embodiment, the Cas12b protein is selected from a group consisting of AacCas12b (Alicyclobacillus acidoterrestris), Aac2Cas12b (Alicyclobacillus acidiphilus), AkCas12b (Alicyclobacillus kakegawensis), AmCas12b (Alicyclobacillus macrosporangiidus), AhCas12b (Alicyclobacillus herbarius), and AcCas12b (Alicyclobacillus contaminans).

In another preferred embodiment, the nucleic acid probe comprises a single-stranded DNA carrying a detectable label.

In another preferred embodiment, the single-stranded DNA is a fluorescent-labeled and biotin-labeled single-stranded DNA.

In another preferred embodiment, the single-stranded DNA is a fluorescent-labeled single-stranded DNA.

In another preferred embodiment, the single-stranded DNA is a fluorescent probe that is labeled with a fluorescent group HEX at a 5' terminal and labeled with a quenching group BHQ1 at a 3' terminal.

In a fifth aspect of the present invention, provided is a kit for detecting a target nucleic acid molecule, the kit including:
i) a first container and a Cas protein in the first container, the Cas protein being Cas12a or a Cas protein having a collateral single-stranded DNA cleavage activity similar to that of the Cas12a;
ii) an optional second container and a guide RNA in the second container, the guide RNA guiding the Cas protein to specifically bind to the target nucleic acid molecule;
iii) a third container and a nucleic acid probe in the third container; and
iv) an optional fourth container and a buffer solution located in the fourth container;
   wherein the target nucleic acid molecule is a target DNA.

In another preferred embodiment, any two, three, or four (or all) of the first, second, third, and fourth containers may be the same or different containers.

In another preferred embodiment, the nucleic acid probe carries a fluorescent group and a quenching group.

In a sixth aspect of the present invention, provided is a method for detecting whether a target nucleic acid molecule exists in a sample, including the following steps:
(a) providing the detection system for detecting a target nucleic acid molecule according to the fourth aspect of the present invention, wherein the detection system further has a sample to be detected; and
(b) detecting whether the nucleic acid probe in the detection system is cleaved by a Cas protein, wherein the cleavage is a trans-cleavage of a collateral single-stranded DNA;
   where if the nucleic acid probe is cleaved by the Cas protein, then it indicates the presence of the target nucleic acid molecule in the sample; and if the nucleic acid probe is not cleaved by the Cas protein, then it indicates the absence of the target nucleic acid molecule in the sample.

In another preferred embodiment, the sample to be detected includes an un-amplified sample and an amplified (or nucleic-acid-amplified) sample.

In another preferred embodiment, the sample to be detected is a sample obtained by amplification.

In another preferred embodiment, a method for amplifying the nucleic acid is selected from a group consisting of PCR amplification, LAMP amplification, RPA amplification, ligase chain reaction, branched DNA amplification, NASBA, SDA, transcription-mediated amplification, rolling circle amplification, HDA, SPIA, NEAR, TMA, and SMAP2.

In another preferred embodiment, the PCR includes high temperature PCR, normal-temperature PCR, and low-temperature PCR.

In another preferred embodiment, the method is used for detecting whether SNP, point mutation, deletion, and/or insertion exists in a nucleic acid at a target site.

In another preferred embodiment, when the upstream and downstream (in a range of -20 nt to +20 nt, preferably in a range of -15 nt to +15 nt, and more preferably in a range of -10 nt to +10 nt) of the target site lack a PAM sequence, nucleic acid amplification is carried out using a PAM-introducing primer.

In another preferred embodiment, the PAM-introducing primer has a structure of formula I in 5'-3':

P1-P2-P3 (I)

wherein,
P1 is a 5' segment sequence that is located at the 5' terminal and is complementary or non-complementary to the sequence of the target nucleic acid molecule;
P2 is a PAM sequence;
P3 is a 3' segment sequence that is located at the 3' terminal and is complementary to the sequence of the target nucleic acid molecule.

In another preferred embodiment, the PAM primer specifically binds to upstream or downstream of the target nucleic acid molecule.

In another preferred embodiment, P1 has a length of 0-20 nt.

In another preferred embodiment, P3 has a length of 5-20 nt.

In another preferred embodiment, the PAM primer has a length of 18-50 nt, and preferably 20-35 nt.

In another preferred embodiment, the complementation includes complete complementation and partial complementation.

In another preferred embodiment, at least one primer containing the PAM sequence is used in the nucleic acid amplification.

In another preferred embodiment, when the upstream and downstream (in a range of -20 nt to +20 nt, preferably in a range of -15 nt to +15 nt, and more preferably in a range of -10 nt to +10 nt) of the target site contain a PAM sequence, then a primer containing or not containing the PAM sequence can be used, and the amplified amplification product contains the PAM sequence.

In another preferred embodiment, the detection in step (b) includes a fluorescence detection method.

In another preferred embodiment, the fluorescence detection method uses a microplate reader or a fluorescence spectrophotometer for detection.

In a seventh aspect of the present invention, provided is use of a Cas protein in preparation of a detection reagent or kit for detecting a target nucleic acid molecule based on collateral single-stranded DNA cleavage, where the Cas protein is Cas12a or a Cas protein having a collateral single-stranded DNA cleavage activity similar to that of the Cas12a.

In another preferred embodiment, the Casl2a is selected from a group consisting of FnCas12a, AsCas12a, LbCas12a, Lb5Cas12a, HkCas12a, OsCas12a, TsCas12a, BbCas12a, BoCas12a, Lb4Cas12a, or a combination thereof; and more preferably, the Cas12a is LbCas12a.

In another preferred embodiment, the Cas protein having a collateral single-stranded DNA cleavage activity similar to that of Cas12a is selected from a group consisting of Cas12b (or C2c1).

In another preferred embodiment, the Cas12b protein is selected from a group consisting of AacCas12b.

It should be understood that, within the scope of the present invention, the above-mentioned technical features of the present invention and the technical features described in detail hereafter (e.g., in examples) can be combined with each other to form a new or preferred technical solution. As limited by the length, it will not be repeated any more here.

### BRIEF DESCRIPTION OF DRAWINGS

FIG. 1 shows a *cis* cleavage characteristic of Cas12a in cleaving a target single-stranded DNA.
FIG. 2 shows that when cleaving a target single-stranded DNA, Cas12a does not depend on a PAM sequence required for cleaving double strands.
FIG. 3 shows a *trans* cleavage characteristic of Cas12a in cleaving a single-stranded DNA.
FIG. 4 shows test Cas12as from 10 different sources, where all of these Cas12as have *cis-*cleavage and *trans*-cleavage activities on a single-stranded DNA.
FIG. 5 identifies sites possibly related to cis-cleavage and *trans*-cleavage activities on the single-stranded DNA in Cas12a through a single-site mutation experiment of the Cas12a.
FIG. 6 shows the structures of Casl2a and Cas12b (i.e., C2c1) monomers and their complexes with a guide RNA and a target DNA.
FIG. 7 shows fluorescence values obtained by different Cas12as using a specific double-stranded DNA substrate and a single-stranded DNA (HEX-N12-BHQ1) as a fluorescence detection probe. The negative control group is not added with the specific substrate.
FIG. 8 shows a schematic flow chart of a HOLMES method for detecting a target DNA based on target DNA amplification and the *trans*-cleavage activity of the Cas12a on a collateral single-stranded DNA.
FIG. 9 shows a sensitivity test of a target DNA by using FnCas12a or LbCas12a directly, or in combination with the HOLMES method.
FIG. 10 shows fluorescence detection values of target sequences having different single-point mutations as detected by the HOLMES method using crRNAs of different lengths of guide sequences in combination with FnCas12a or LbCas12a.
FIG. 11 tests whether a FAM-labeled single-stranded DNA probe is trans-cleaved after the target single-stranded DNA is added by utilizing a FAM-labeled fluorescent probe and 10 Casl2a proteins.
FIG. 12 tests fluorescence values after the target single-stranded DNA is added by using HEX-N12-BHQ1 as a probe and 10 Cas12a proteins.
FIG. 13 (A) shows HOLMES detection values when a gyrB gene fragment is used as a target sequence and different concentrations of pure cultured *Escherichia coli* MG1655 are used as positive control templates by using a single-stranded DNA fluorescent probe labeled with HEX and BHQ1 at two ends thereof. It is shown that the fluorescence response value of *Escherichia coli* MG1655 decreases with the decrease of its concentration. (B) Detection values of water samples in environments at different locations.
FIG. 14 shows a schematic flow chart of a HOLMES method for detecting SNP, and fluorescence detection values of 5 SNP sites.
FIG. 15 shows fluorescence detection values of key sites in a TP53 gene (a cancer-related gene) as detected by the HOLMES method.
FIG. 16 shows detection values of 5 SNP sites (related to gout) as detected by the HOLMES method.
FIG. 17 shows detection values of one SNP site (related to gout) as detected by the HOLMES method, where the samples are samples from 21 volunteers.
FIG. 18 shows a primer design scheme of one example of the present invention, which can be used for HOLMES detection of SNP at any site.

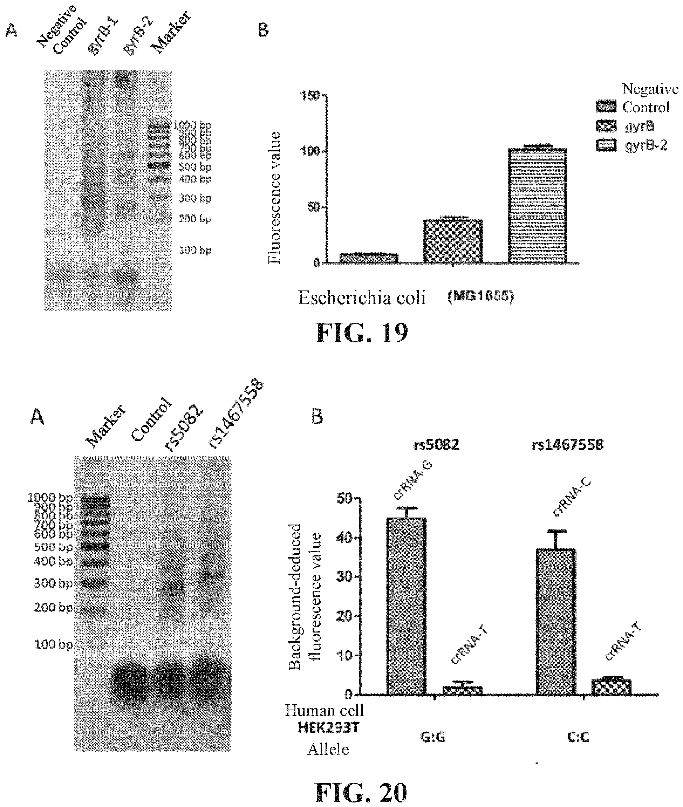

FIG. 19 uses a combination of LAMP and HOLMES to detect *Escherichia coli* in the system. (A) An electrophoresis map of a gyrB gene of *Escherichia coli* amplified by LAMP. A total of two sets of primers gyrB-1 and gyrB-2 are used for amplification. gyrB is the characteristic gene of *Escherichia coli.* (B) A HOLMES detection system is used for detecting an amplification product of LAMP. Negative control: the sample is sterile water, and a gyrB-1 amplification primer is used for amplifying or detecting the result of the gyrB gene; gyrB-1: the sample is *Escherichia coli* to be detected, and a first set of gyrB gene amplification primers are used for amplifying or detecting the result of the gyrB gene; and gyrB-2: the sample is *Escherichia coli* to be detected, and a second set of gyrB gene amplification primers are used for amplifying or detecting the result of the gyrB gene.
FIG. 20 detects the genotype of a human HEK293T cell by using a combination of LAMP and HOLMES. (A) An electrophoresis map of a corresponding SNP detection template of the human HEK293T cell as amplified by LAMP. Negative control: the sample is sterile water, and the result is a result of amplification using an rs5082 amplification primer; rs5082: the sample is a total genome of the human HEK293T cell, and the result is a result of amplification using the rs5082 amplification primer; and rs1467558: the sample is the total genome of the human HEK293T cell, and the result is a result of amplification using an rs1467558 amplification primer. (B) A HOLMES detection system is used for detecting an amplification product of LAMP. The rs5082 site was detected using two crRNAs of crRNA-G and crRNA-T respectively (Sequence Listing 5); and the rs1467558 site was detected using two crRNAs of crRNA-C and crRNA-T respectively (Sequence Listing 5).

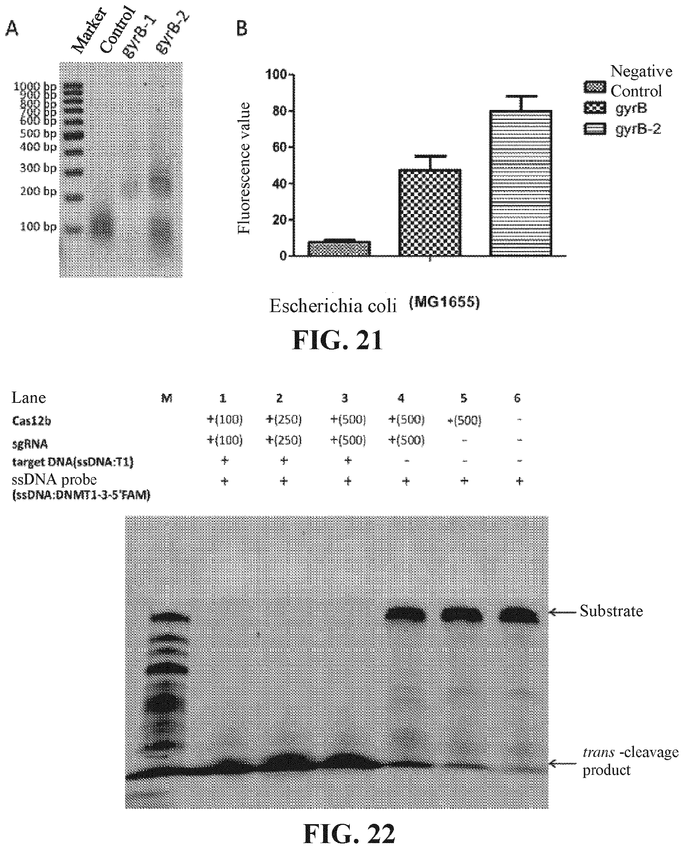

FIG. 21 uses a combination of RPA and HOLMES to detect *Escherichia coli* in the system. (A) Amplification of the gyrB gene of *Escherichia coli* by RPA. A total of two sets of primers gyrB-1 and gyrB-2 are used for amplification. gyrB is a characteristic gene of *Escherichia coli.* (B) A HOLMES detection system is used for detecting an amplification product of RPA. Negative control: the sample is sterile water, and a gyrB-1 amplification primer is used for amplifying or detecting the result of the gyrB gene; gyrB-1: the sample is *Escherichia coli* to be detected, and a first set of gyrB amplification primers are used for amplifying or detecting the result of the gyrB gene; and gyrB-2: the sample is *Escherichia coli* to be detected, and a second set of gyrB amplification primers are used for amplifying or detecting the result of the gyrB gene.
FIG. 22 shows detection of collateral single-stranded DNA cleavage activity of Cas12b when a single-stranded DNA is used as the target DNA. After the collateral cleavage reaction is completed, the reactants are separated by 12% urea-denatured gel electrophoresis, and detected by a fluorescence imaging system. The numbers in brackets represent the final concentrations of the reactants in nM; the target DNA is a 66 nt long single-stranded DNA at a dosage of 50 nM; and the single-stranded DNA probe is a single-stranded DNA carrying a FAM label at the 5' terminal at a dosage of 50 nM. As can be seen from the figure, after Cas12b, the guide RNA and the target DNA are contained, the FAM-labeled single-stranded DNA is cut into fragments, i.e., Cas12b has a collateral single-stranded DNA cleavage activity.

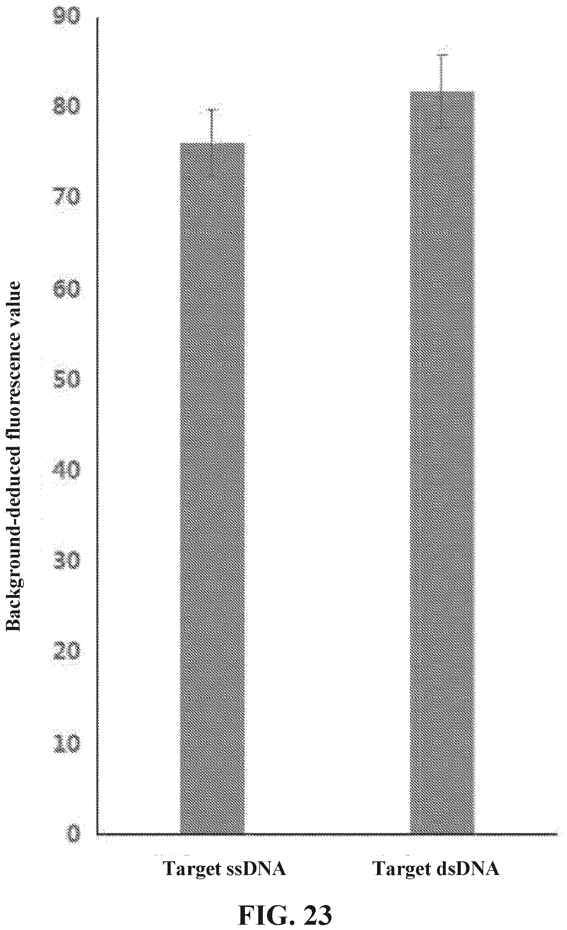

FIG. 23 shows detection of collateral single-stranded DNA cleavage activity of Cas12b when a single-stranded DNA and a double-stranded DNA are used as the target DNAs. After the collateral cleavage reaction is completed, the reactants are detected by using a fluorescence microplate reader. The dosages of Cas12b and the guide RNA are both 500 nM; the target DNA is a 66 nt long single-stranded DNA or double-stranded DNA at a dosage of 50 nM; and the single-stranded DNA probe is a single-stranded DNA probe (HEX-N12-BHQ1) containing a fluorescence report group and a quenching group at a dosage of 500 nM. As can be seen from the figure, regardless of whether a single-stranded DNA template or a double-stranded DNA template is used, the collateral single-stranded DNA cleavage activity can be detected after Cas12b and the guide RNA are added.
FIG. 24 shows the collateral single-stranded DNA *trans*-cleavage activity of Cas12b on a target DNA at a low concentration after the combination with LAMP amplification.

### DETAILED DESCRIPTION

In order to make the objectives, technical solutions and advantages of the embodiments of the present invention clearer, the following clearly and completely describes the technical solutions in the embodiments of the present invention with reference to the accompanying drawings in the embodiments of the present invention. The described embodiments are a part rather than all of the embodiments of the present invention. All other embodiments obtained by a person of ordinary skill in the art based on the embodiments of the present invention without creative efforts fall within the protection scope of the present invention.

The inventor has developed a technical solution for target nucleic acid detection through extensive and in-depth research and through research on the cleavage characteristics of a Cas enzyme (such as Cas12a and Cas12b enzymes). The experimental results show that a nucleic acid is detected successfully and rapidly by employing the above-mentioned technical solution, for example, for the identification of whether there is a certain concentration of microorganisms such as *Escherichia coli* in water, and for the rapid identification of an SNP genotype. The present invention is completed on this basis.

### Terms

The term "guide RNA" refers to an RNA that guides a Cas protein to specifically bind to a target DNA sequence.

The term "crRNA" refers to a CRISPR RNA, which is a short RNA that guides Cas12a to bind to a target DNA sequence.

The term "CRISPR" refers to a clustered regularly interspaced short palindromic repeat, which is the immune system of many prokaryotes.

The term "Cas protein" refers to a CRISPR-associated protein, which is a related protein in a CRISPR system.

The term "Cas12a" (formerly referred to as "Cpf1") refers to a crRNA-dependent endonuclease, which is a type V-A enzyme in CRISPR system classification.

The terms "Cas12b" and "C2c1" are used interchangeably, and refer to a crRNA-dependent endonuclease, which is a type V-B enzyme in CRISPR system classification.

The term "LAMP" is a Loop-mediated isothermal amplification technology, which is an isothermal nucleic acid amplification technology suitable for gene diagnosis.

The term "PAM" refers to a protospacer-adjacent motif, which is necessary for Cas12a cleavage. The PAM of FnCas12a is a TTN sequence, the PAM of LbCas12a is a TTTN sequence, and the PAM of AacCas12b is TTN.

The present invention discloses a method for detecting a target nucleic acid molecule, which includes: adding a guide RNA, a Cas protein, a nucleic acid probe and a buffer solution into a reaction system containing a target nucleic acid molecule to be detected, and then performing fluorescence detection of the target nucleic acid molecule.

The Cas protein is Cas12a or Cas12b.

The Cas12a is preferably one of FnCas12a, AsCas12a, LbCas12a, Lb5Cas12a, HkCas12a, OsCas12a, TsCas12a, BbCas12a, BoCas12a or Lb4Cas12a; and the Cas12a is preferably LbCas12a.

The Cas12b is preferably AacCas12b, Aac2Cas12b, AkCas12b, AmCas12b, AhCas12b or AcCas12b.

A guide RNA refers to an RNA that guides a Cas protein to specifically target a DNA sequence.

The target nucleic acid molecule to be detected in the reaction system of the target nucleic acid molecule to be detected is obtained by amplification.

The detection method can detect a pathogenic microorganism, gene mutation, or a specific target DNA.

Use of a Cas protein in a method for detecting a target nucleic acid molecule.

When a target DNA, a guide RNA and a Cas protein form a ternary complex, the complex will cleave other single-stranded DNA molecules in the system.

A guide RNA refers to an RNA that guides a Cas protein to specifically target a DNA sequence.

The present invention also provides a kit including a guide RNA, a Cas protein, and a nucleic acid probe. Furthermore, the kit of the present invention can also include a buffer solution.

The present invention provides a detection method for rapidly detecting a target nucleic acid molecule with high specificity. Once the (single-stranded or double-stranded) target DNA, the crRNA and the Cas12a protein form a ternary complex, the complex will cleave other single-stranded DNA molecules in the system. Through designing, the crRNA targets the target DNA (a segment of DNA sequence to be detected); the crRNA and the Cas12a protein are added into the detection system; when the target DNA is present, the Cas12a forms a ternary complex with the crRNA and the target DNA, and meanwhile, the complex exerts its collateral cleavage activity and cleaves a single-stranded DNA labeled with a fluorescent signal (two ends of the single-stranded DNA are respectively connected with a luminescent group and a quenching group, and the luminescent group can emit light after being cleaved), thereby emitting fluorescence. Therefore, whether the system to be detected contains the target DNA molecule can be known by fluorescence detection. By using the method of the present invention, whether a specific DNA sequence is contained in a sample can be quickly detected. The sensitivity of the detection method can be greatly improved by combining with a PCR technology. The nucleic acid probe in the present invention is preferably a fluorescent probe.

### HOLMES condition testing:

The present invention provides use of Cas12 enzymes such as Cas12a and Cas12b in nucleic acid detection. The following description will take Cas12a as an example.

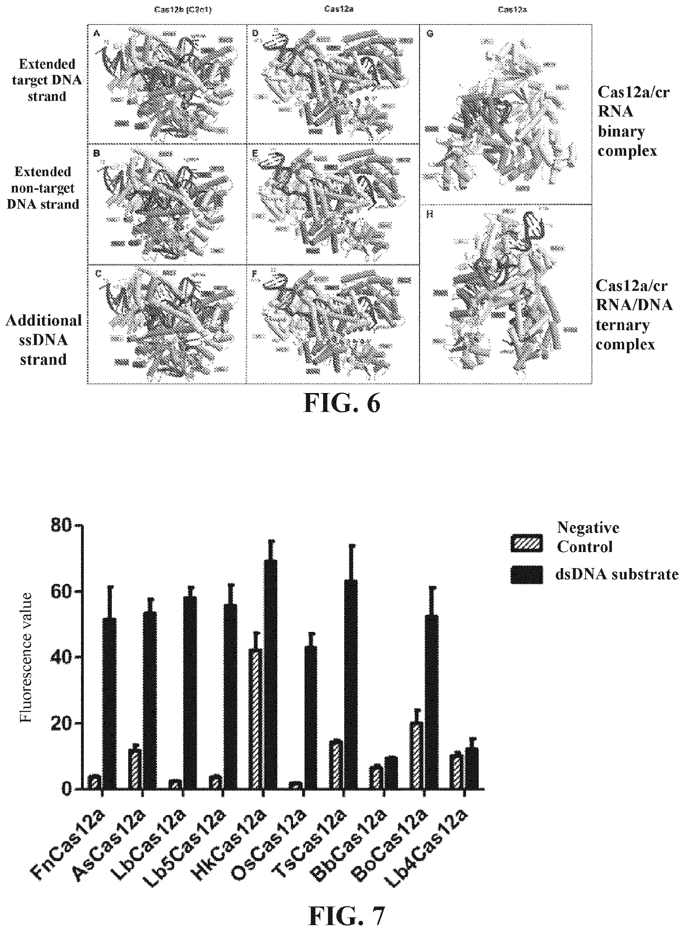

**Selection of Cas12a**: according to research, Cas12a has a trans-cleavage activity, that is, once the target DNA, the crRNA and the Cas12a protein form a ternary complex, other single-stranded DNAs (collateral single-stranded DNAs) in the system will be cleaved. According to this principle, a specific DNA detection method is designed. First, the collateral DNA is designed as a fluorescent probe, which consists of a random sequence of 12 nt, and is labeled with a fluorescent group HEX at the 5' terminal and a quenching group BHQ1 (HEX-N12-BHQ1) at the 3' terminal. When the target DNA fragment is contained in the system, a ternary complex of the target DNA, the crRNA and the Casl2a protein will be formed. At this time, the probe will be cleaved, and meanwhile, the HEX fluorescent group will emit fluorescence (with an excitation light at 535 nM and an emission light at 556 nM) as detected by a fluorescence detector. Next, 10 different Cas12as are tested, and the target sequence is a double-stranded DNA as shown in FIG. 7. It can be seen that the complex composed of the target double-stranded DNA and each Cas12a protein can realize the *trans* cleavage activity.

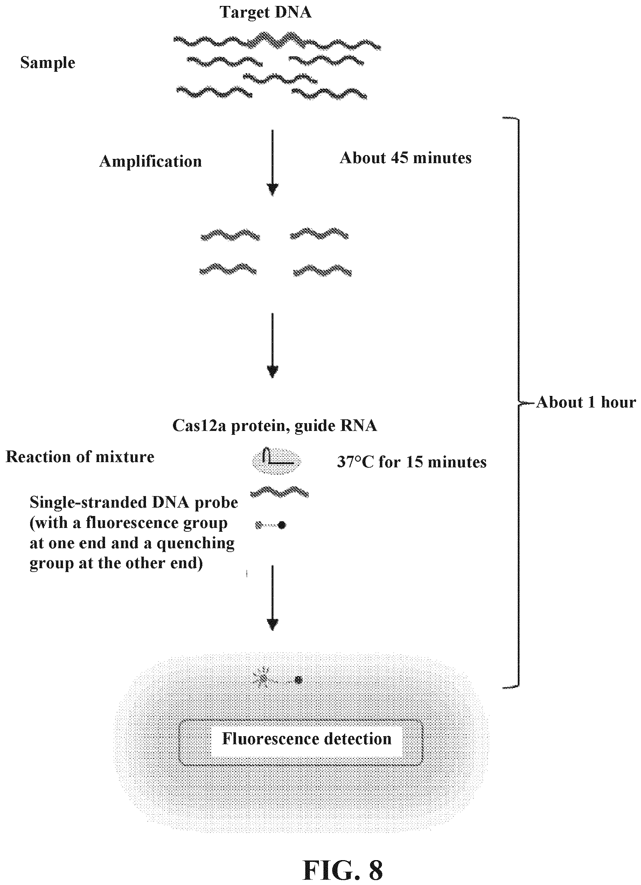

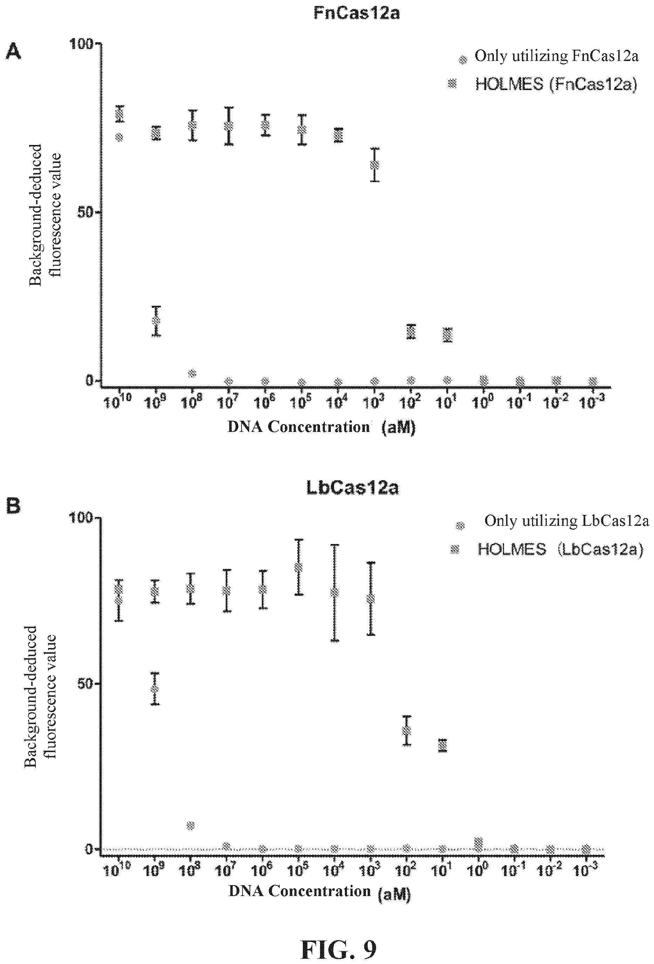

**HOLMES response sensitivity:** then, the response sensitivities of FnCas12a and LbCas12a to the target DNA are tested, that is to say, the lowest concentration of the target DNA at which the response can occur is investigated. As shown in FIG. 9, when the test target is directly added, they can respond to the target DNA with a concentration above 0.1 nM, and the response is remarkable when the concentration is above 1 nM. If a PCR technology (the HOLMES method) is combined, as shown in FIG. 8, i.e., amplification of the fragment of interest through PCR followed by a Cas12a cleavage reaction, the response sensitivity can be as low as 10 aM, as shown in FIG. 9.

**SNP Test:** next, whether the HOLMES method can detect an SNP genotype is tested. T1 is taken as a target sequence, PAM at this site is mutated or positions 1-18 of the target sequence are respectively subjected to single-point mutation, and the detection differences between a non-mutated sequence and a mutated sequence by crRNAs of different lengths are compared.

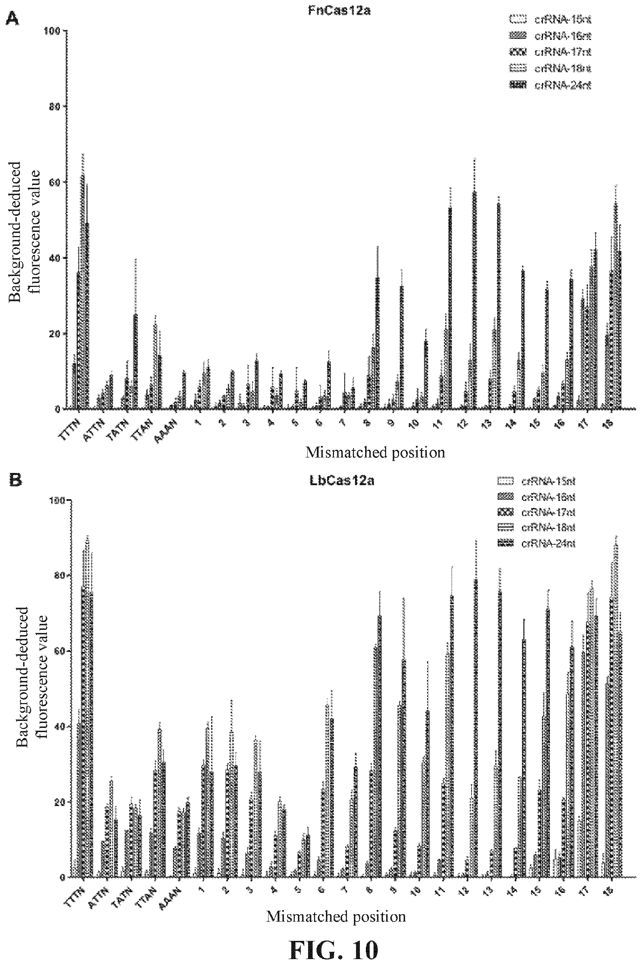

As shown in FIG. 10, when the complementary sequence of the target is a crRNA of 24 nt (crRNA-24nt), the single-point mutation at the positions 8-18 is not much different from the wild type, while the fluorescence value decreases obviously after the PAM mutation and the mutation of positions 1-7. When the crRNA is truncated and the length of a paired target sequence is 18 nt, the fluorescence value of the mutation positions of 8-16 nt is obviously decreased compared with that of a target sequence having a length of 24 nt; when the length of the crRNA continues to be shortened to 16 nt or 17 nt, the fluorescence value of the mutated target sequence decreases more significantly; and when it is further shortened to 15 nt, the fluorescence value of the mutated target sequence is weaker as compared with that of this target sequence, but the fluorescence intensity of the mutated target sequence may still be higher as compared with those of other target sequences, and thus can be used for detection. Taken together, the crRNAs of 15 nt, 16 nt and 17 nt are the most suitable for SNP detection.

In the present invention, the Cas12a cleaves the single-stranded DNA in a PAM-sequence independent programmed cleaving manner, which is called *cis* cleavage; but once the ternary complex Cas12a/crRNA/target DNA is formed, it will show a *trans*-cleavage activity, i.e., exhibiting an activity of cleaving any non-target single-stranded DNA in the system.

Based on the characteristics of Cas12a, a method for specific nucleic acid molecule detection is developed, which is called HOLMES (one HOur Low-cost Multipurpose Efficient Simple assay). Just like the name of the technology, it is characterized by a fast speed (1 hour), a low price, multiple channels, high efficiency and a simple test method. The method can be used in the fields of rapid pathogen detection, SNP detection and the like.

### Nucleic acid detection based on the collateral cleavage activity

The present invention also provides a nucleic acid detection method based on the collateral cleavage activity of the Cas12 enzyme (including Cas12a or Cas12b).

Preferably, the detection of the present invention can be performed for SNP, and in particular PCR amplification is performed first followed by detection.

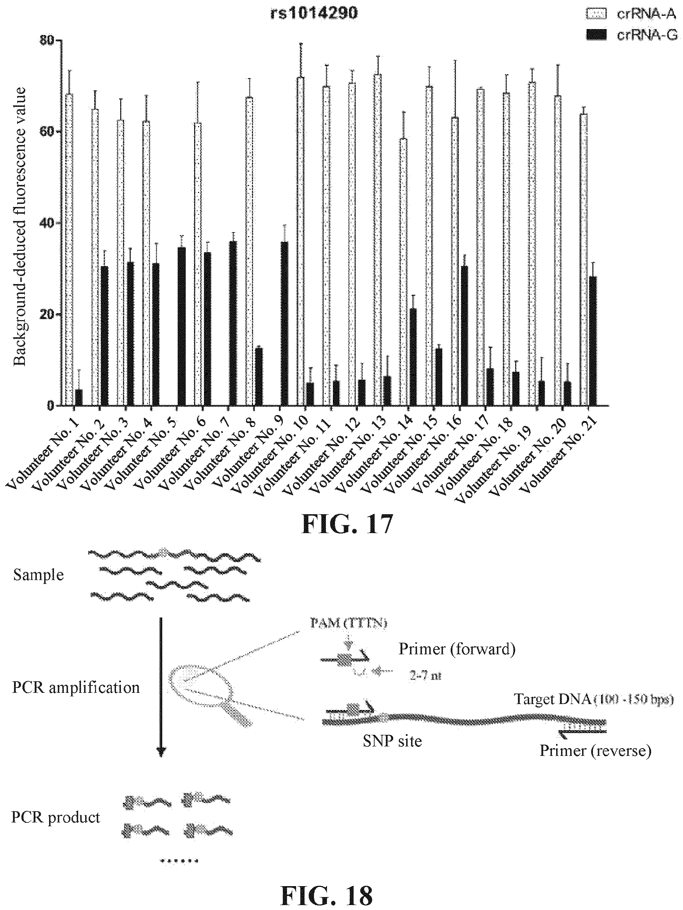

Referring to FIG. 18, a primer design scheme is given.

Case 1. When there is a PAM site near the SNP site, and a crRNA synthesized based on a guide sequence designed according to the PAM site can be used for HOLMES detection. When the HOLMES method is used for detection, it shows a relatively low background signal; and for the same guide sequence, signal differences between different SNP templates are quite large.

Case 2. When there is no PAM site or no suitable PAM site near the SNP site, a PAM site can be introduced according to the above experimental scheme.

A typical step includes designing a primer near the SNP site, and carrying a PAM site on the primer, where the sequence located at the 3' terminal of the PAM site should be paired with the template DNA. There is no special requirement on the primer at the other terminal, as long as the primer can be paired with template DNA and can be used for PCR amplification. As shown in FIG. 18, PAM sites can be successfully introduced after PCR amplification.

Referring to FIG. 10, in the present invention, when designing introduction of the PAM site, the SNP site is usually located at the first 16 bases of the 5' terminal of the crRNA guide sequence, preferably at positions 1-14, more preferably at positions 1-12, still more preferably at positions 1-11 or 1-10, and most preferably at positions 1-8 or 1-7.

The present invention has the following main advantages:
(1) Fast speed: when the test conditions are ready, it only takes about 1 hour from the time of obtaining a sample to the time of obtaining test results.
(2) Low cost: there are no special materials or enzymes in the experiment, and it involves a small quantity of materials and reagents, and thus it can be used for trace analysis.
(3) High efficiency: the present invention has extremely high sensitivity and can detect DNA at a concentration of 10 aM.
(4) Multiple uses: it can detect different nucleic acid samples, including DNA samples and RNA samples.
(5) Simplicity: there are no special or complicated steps, and if a kit is prepared and a program is set, only simple operations such as adding a sample are required.

The present invention will be described in detail below in connection with specific examples. It should be understood that the following examples are only intended to illustrate the present invention, rather than limiting the scope of the present invention. The experimental methods in the following examples which are not specified with specific conditions are generally carried out according to conventional conditions, such as those described in Sambrook et al., Molecular Cloning: Laboratory Manual (New York: Cold Spring Harbor Laboratory Press, 1989), or those recommended by manufacturers. Unless otherwise stated, percentages and parts are weight percentages and parts by weight.

Unless otherwise stated specifically, the experimental materials involved in the present invention can be obtained from commercial channels.

### Materials

1. The RNase inhibitor is purchased from TaKaRa, and the high-fidelity DNA polymerase KOD FX is purchased from ToYoBo; the primers (oligonucleotides) are synthesized by Sangon Biotech (Shanghai) Co., Ltd.; the T7 RNA polymerase is purchased from Thermo; the RNA purification and concentration kit (RNA Clean&ConcentratorTM_5) is purchased from Zymo Research; the Wizard® SV Gel and PCR Clean-Up System is purchased from Promega; and the media (e.g., Tryptone, Yeast Extract, etc.) are all purchased from OXOID.
2. Medium formula: a liquid LB (1% Tryptone, 0.5% Yeast extract, 1% NaCl), and only 2% agar needs to be added into the liquid LB when a solid LB is prepared.

### Example 1 Cas12a protein detection capable of detecting a single-stranded DNA target (the probe being labeled with FAM)

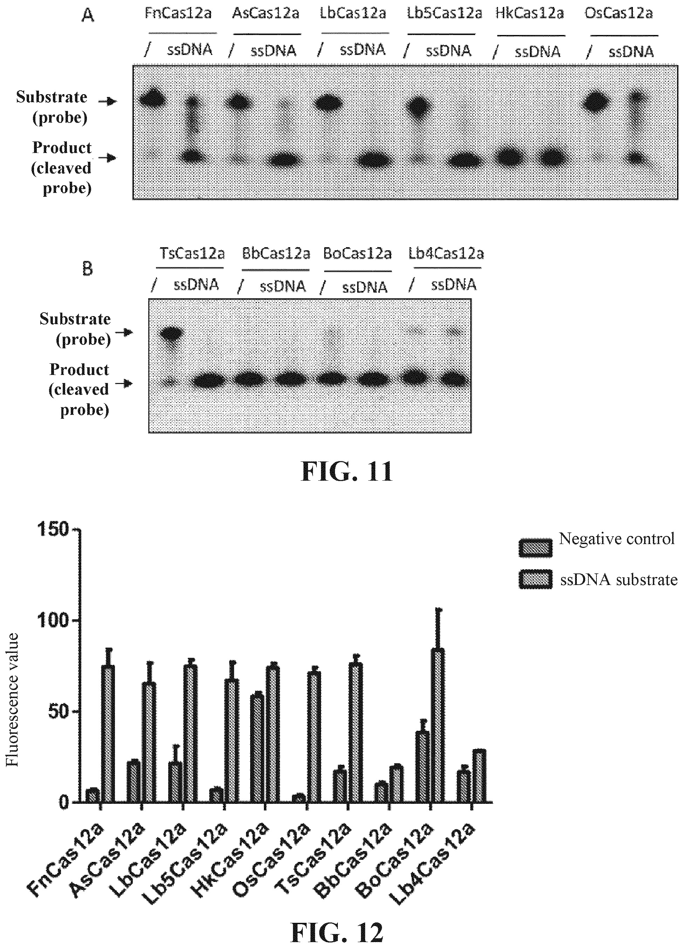

A single-stranded DNA (target-T1-R) was selected as a target sequence to test the response values of its detection by different Cas12a proteins.
1. Preparation of crRNA: first, a transcription template was prepared by annealing T7-crRNA-F to a synthesized oligonucleotide T7-T1-24-R as shown in Table 5. Specifically, the paired oligonucleotide (4 µM) was annealed in a 1X PCR buffer (Transgen Biotech) at a total volume of 50 µL, and then subjected to an annealing procedure: initially denaturing at 95°C for 5 minutes, and then cooling from 95°C to 20°C at a rate of 1°C per minute using a thermal cycler. crRNA was synthesized using a T7 high-throughput transcription kit, and the reaction was carried out overnight at 37°C (for about 16 h). Then RNA was purified using the RNA purification and concentration kit, quantified with NanoDrop 2000C (Thermo Fisher Scientific), diluted to a concentration of 10 µM, and stored in a refrigerator at -80°C.
2. Cas12a reaction: a 20 µL reaction system was added with the crRNA (0.5 µM) purified in step 1, a Cas12a (0.25 µM), a target single-stranded DNA (target-T1-R) (0.01 µM), a nucleic acid probe (N25-5' FAM) (0.01 µM), a buffer solution of NEB buffer 3.1, and 0.5 µL of an RNase inhibitor. The blank control reaction was a reaction in which all other components were added except the single-stranded DNA target sequence. Reaction was conducted at 37°C for 15 min, and then terminated at 98°C for 2 min.
3. Fluorescence detection: the reaction was subjected to urea-acrylamide gel electrophoresis (Urea-PAGE), and then detected with a fluorescence luminescence imager. As shown in FIG. 11, different Cas12as have different detection effects on the target. For example, for HkCas12a, etc., cleavage of the probe was caused even when no target single-stranded DNA was added. LbCas12a and the like are better candidates of Cas12a proteins because the cleavage of the probe occurred only when the target single-stranded DNA was added.

### Example 2 Cas12a protein detection capable of detecting a single-stranded DNA target (the probe being labelled with two labels of HEX and BHQ1)

A single-stranded DNA (target-T1-R) was selected as a target sequence to test the response values of its detection by different Cas12a proteins.
1. Preparation of crRNA: first, a transcription template was prepared by annealing T7-crRNA-F to a synthesized oligonucleotide T7-T1-24-R (Table 5). Specifically, the paired oligonucleotide (4 µM) was annealed in a 1X PCR buffer (Transgen Biotech) at a total volume of 50 µL, and then subjected to an annealing procedure: initially denaturing at 95°C for 5 minutes, and then cooling from 95°C to 20°C at a rate of 1°C per minute using a thermal cycler. crRNA was synthesized using a T7 high-throughput transcription kit, and the reaction was carried out overnight at 37°C (for about 16 h). Then RNA was purified using the RNA purification and concentration kit, quantified with NanoDrop 2000C, diluted to a concentration of 10 µM, and stored in a refrigerator at -80°C.
**2.** Casl2a reaction: a 20 µL reaction system was added with the crRNA (0.5 µM) purified in step 1, a Cas12a (0.25 µM), a target single-stranded DNA (target-T1-R) (0.01 µM), a fluorescence probe (HEX-N12-BHQ1, i.e., a 12 nt single-stranded DNA that is labeled with HEX at the 5' terminal and with the BHQ1 at the 3' terminal) (0.5 µM), a buffer solution of NEB buffer 3.1, and 0.5 µL of an RNase inhibitor. The control reaction was a reaction in which all other components were added except the single-stranded DNA target sequence. Reaction was conducted at 37°C for 15 min, and then terminated at 98°C for 2 min.
3. Fluorescence detection: 20 µL of the inactivated reaction solution was added into a 96-well plate, and then detected by a microplate reader (with an excitation light at 535 nm and an emission light at 556 nm). As shown in FIG. 12, different Cas12as have different detection effects on the target. For example, for HkCas12a, etc., cleavage of the probe was caused even when no target single-stranded DNA was added. FnCas12a and the like are better candidates of Cas12a proteins because the cleavage of the probe occurred only when the target single-stranded DNA was added.

### Example 3 Cas12a protein detection capable of detecting a double-stranded DNA target

A double-strand DNA (target-T1) was selected as a target sequence to test the response values of its detection by different Cas12a proteins.
1. Preparation of crRNA: first, a transcription template was prepared by annealing T7-crRNA-F to a synthesized oligonucleotide T7-T1-24-R (Table 5). Specifically, the paired oligonucleotide (4 µM) was annealed in a 1X PCR buffer (Transgen Biotech) at a total volume of 50 µL, and then subjected to an annealing procedure: initially denaturing at 95°C for 5 minutes, and then cooling from 95°C to 20°C at a rate of 1°C per minute using a thermal cycler. crRNA was synthesized using a T7 high-throughput transcription kit, and the reaction was carried out overnight at 37°C (for about 16 h). Then RNA was purified using the RNA purification and concentration kit, quantified with NanoDrop 2000C, diluted to a concentration of 10 µM, and stored in a refrigerator at -80°C.
2. Cas12a reaction: a 20 µL reaction system was added with the crRNA (0.5 µM) purified in step 1, a Casl2a (0.25 µM), a target double-stranded DNA (target-T1, obtained by annealing primers target-T1-F to target-T1-R) (0.01 µM), a fluorescence probe (HEX-N12-BHQ1) (0.5 µM), a buffer solution of NEB buffer 3.1, and 0.5 µL of an RNase inhibitor. Reaction was conducted at 37°C for 15 min, and then terminated at 98°C for 2 min.
3. Fluorescence detection: 20 µL of the inactivated reaction solution was added into a 96-well plate, and then detected by a microplate reader (with an excitation light at 535 nm and an emission light at 556 nm). As shown in FIG. 7, different Cas12as have different detection effects on the target. LbCas12a and the like are better candidates of Cas12a proteins because the cleavage of the probe occurred only when the target double-stranded DNA was added.

### Example 4 Testing of different concentrations of the target with FnCas12a and LbCas12a

target-T1 was selected as the target DNA, and then diluted to different concentrations at a gradient to test the response sensitivity of FnCas12a and LbCas12a thereto. In order to increase the sensitivity, a PCR amplification step was added.
1. Preparation of crRNA: first, a transcription template was prepared by annealing T7-crRNA-F to a synthesized oligonucleotide T7-T1-24-R (Table 5). Specifically, the paired oligonucleotide (4 µM) was annealed in a 1X PCR buffer (Transgen Biotech) at a total volume of 50 µL, and then subjected to an annealing procedure: initially denaturing at 95°C for 5 minutes, and then cooling from 95°C to 20°C at a rate of 1°C per minute using a thermal cycler. crRNA was synthesized using a T7 high-throughput transcription kit, and the reaction was carried out overnight at 37°C (for about 16 h). Then RNA was purified using the RNA purification and concentration kit, quantified with NanoDrop 2000C, diluted to a concentration of 10 µM, and stored in a refrigerator at -80°C.
2. PCR amplification (optional): a plasmid containing the target target-Tl (pUC18-T1) was used as the template, diluted at a gradient, and then used for the PCR reaction. The total volume of each reaction system was 20 µL, 0.25 µM of M13F-47 and M13R-48 each were used as primers (Table 4), and a high fidelity enzyme KOD FX (ToYoBo) was used for the PCR reaction. The PCR reaction procedure was 95°C for 2 min, and then starting 35 cycles of 98°C for 10 s, 60°C for 15 s, and 68°C for 10 s. After completion of the PCR, the PCR amplification product was directly used for a Casl2a reaction.
3. Casl2a reaction: a 20 µL reaction system was added with the crRNA (0.5 µM) purified in step 1, FnCas12a or LbCas12a (0.25 µM), 1 µL of a PCR product (or target DNAs that are directly diluted into different concentrations), a fluorescent probe (HEX-N12-BHQ1) (0.5 µM), a buffer solution of NEB buffer 3.1, and 0.5 µL of a RNase inhibitor. Reaction was conducted at 37°C for 15 min, and then terminated at 98°C for 2 min.
4. Fluorescence detection: 20 µL of the inactivated reaction solution was added into a 96-well plate, and then detected by a microplate reader (with an excitation light at 535 nm and an emission light at 556 nm). As shown in FIG. 9, when the test target was directly added, all the target DNAs with a concentration above 0.1 nM could respond, and the response was remarkable when the concentration was above 1 nM. If a PCR technology was combined, i.e., amplification of the fragment of interest through PCR followed by a Cas12a cleavage reaction, the response sensitivity could be as low as 10 aM.

### Example 5 Testing of single-point mutant target with FnCas12a and LbCas12a

target-Tl was selected as the target, and it was subjected to single-point mutation in a PAM region and positions 1-18 respectively, so as to test response values of several crRNAs of different lengths to the wild type and the same after single-point mutation.
1. Preparation of crRNA: first, a transcription template was prepared by respectively annealing T7-crRNA-F to synthesized oligonucleotides T7-T1-24-R, T7-T1-15-R, T7-T1-16-R, T7-T1-17-R, and T7-T1-18-R (Table 5). Specifically, the paired oligonucleotide (4 µM) was annealed in a 1X PCR buffer (Transgen Biotech) at a total volume of 50 µL, and then subjected to an annealing procedure: initially denaturing at 95°C for 5 minutes, and then cooling from 95°C to 20°C at a rate of 1°C per minute using a thermal cycler. crRNA was synthesized using a T7 high-throughput transcription kit, and the reaction was carried out overnight at 37°C (for about 16 h). Then RNA was purified using the RNA purification and concentration kit, quantified with NanoDrop 2000C, diluted to a concentration of 10 µM, and stored in a refrigerator at -80°C.
2. PCR amplification: a plasmid containing the target target-T1 (pUC18-T1) was used as the template. The total volume of each reaction system was 20 µL, 0.25 µM of the primer M13R-48 and respective mutation primers for Target-T1-F each were used (Table 4), and a high fidelity enzyme KOD FX (ToYoBo) was used for the PCR reaction. The PCR reaction procedure was 95°C for 2min, and then starting 35 cycles of 98°C for 10 s, 60°C for 15 s, and 68°C for 10 s. After completion of the PCR, the product was directly used for a Cas12a reaction.
3. Casl2a reaction: a 20 µL reaction system was added with the crRNA (0.5 µM) purified in step 1, FnCas12a or LbCas12a (0.25 µM), 1 µL of a PCR product, a fluorescent probe (HEX-N12-BHQ1) (0.5 µM), a buffer solution of NEB buffer 3.1, and 0.5 µL of a RNase inhibitor. Reaction was conducted at 37°C for 15 min, and then terminated at 98°C for 2 min.
4. Fluorescence detection: 20 µL of the inactivated reaction solution was added into a 96-well plate, and then detected by a microplate reader (with an excitation light at 535 nm and an emission light at 556 nm). As shown in FIG. 10, when the complementary sequence of the target was a crRNA of 24 nt (crRNA-24nt), the single-point mutations at the positions 8-18 were not much different from the wild type, while the fluorescence value decreased obviously after the PAM mutation and the point mutation of positions 1-7. When the crRNA was truncated and the length of a paired target sequence was 18 nt, the fluorescence value of the mutation positions of 8-16 nt was obviously decreased compared with that of 24 nt; when the length was 16 nt or 17 nt, the decrease of the fluorescence value of the mutated target sequence is more obvious; and when the length was 15 nt, the fluorescence values of both the target sequence and the mutated target sequence were very weak, but the fluorescence intensity of the mutated target sequence might still be higher as compared with those of other target sequences, and thus could be used for detection. Taken together, the crRNAs of 15 nt, 16 nt and 17 nt are the most suitable for SNP detection.

### Example 6 Testing of Escherichia coli and the like microorganisms in environmental water

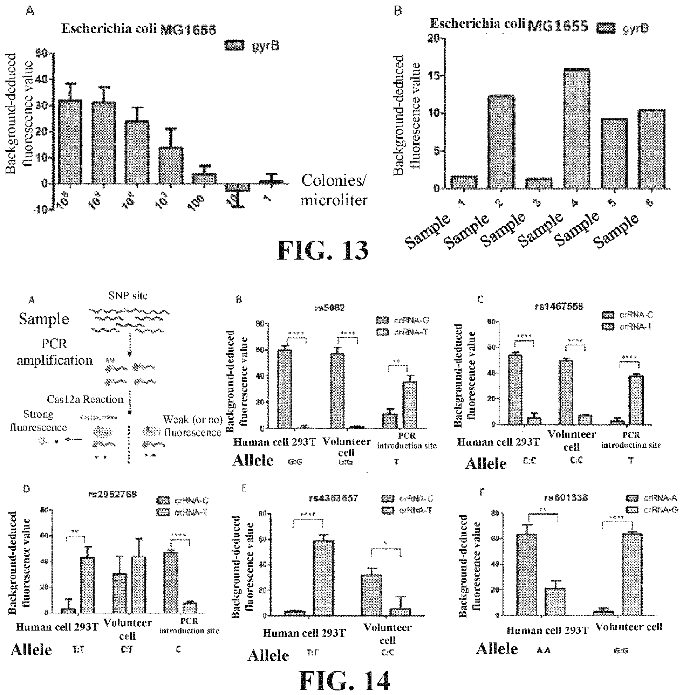

The gyrB gene of *Escherichia coli* was selected as the detection target to indirectly test the concentrations of *Escherichia coli* and the like microorganisms in water. Taking *Escherichia coli* MG1655 as a positive control, the content of microorganisms in water (such as sewage and tap water) in the environment was determined.
1. Preparation of crRNA: first, a transcription template was prepared by annealing T7-crRNA-F to a synthesized oligonucleotide T7-crRNA-gyrB (Table 5). Specifically, the paired oligonucleotide (4 µM) was annealed in a 1X PCR buffer (Transgen Biotech) at a total volume of 50 µL, and then subjected to an annealing procedure: initially denaturing at 95°C for 5 minutes, and then cooling from 95°C to 20°C at a rate of 1°C per minute using a thermal cycler. crRNA was synthesized using a T7 high-throughput transcription kit, and the reaction was carried out overnight at 37°C (for about 16 h). Then RNA was purified using the RNA purification and concentration kit, quantified with NanoDrop 2000C, diluted to a concentration of 10 µM, and stored in a refrigerator at -80°C.
2. PCR amplification: when the positive control sample *Escherichia coli* MG1655 was cultured until the OD₆₀₀ was about 0.5, it was diluted at a 10-fold gradient respectively, and then used as the template, and the sample was ambient water (including tap water and muddy water in the environment). The total volume of each reaction system was 20 µL, 0.25 µM of primers gyrB-F and gyrB-R each were used (Table 4), and a high fidelity enzyme KOD FX (ToYoBo) was used for the PCR reaction. The PCR reaction procedure was 95°C for 2 min, and then starting 35 cycles of 98°C for 10 s, 60°C for 15 s, and 68°C for 10 s. After completion of the PCR, the PCR product was directly used for a Cas12a reaction.
3. Casl2a reaction: a 20 µL reaction system was added with the crRNA (0. 5 µM) purified in step 1, LbCas12a (0.25 µM), 1 µL of a PCR product, a fluorescence probe (HEX-N12-BHQ1) (0.5 µM), a buffer solution of NEB buffer 3.1, and 0.5 µL of an RNase inhibitor. Reaction was conducted at 37°C for 15 min, and then terminated at 98°C for 2 min .
4. Fluorescence detection: 20 µL of the inactivated reaction solution was added into a 96-well plate, and then detected by a microplate reader (with an excitation light at 535 nm and an emission light at 556 nm). As shown in FIG. 13, the fluorescence response value of *Escherichia coli* MG1655 decreases with the concentration decrease. Among them, microorganisms were detected more obviously in samples 2, 4, 5 and 6.

### Example 7 Testing of Human SNP

The SNP test selected 5 sites of human SNP, namely rs5082, rs1467558, rs2952768, rs4363657, and rs601338, to test the feasibility of the HOLMES method.
1. Preparation of crRNA: first, a transcription template was prepared by annealing T7-crRNA-F to a synthesized oligonucleotide (Table 5). Specifically, the paired oligonucleotide (4 µM) was annealed in a 1X PCR buffer (Transgen Biotech) at a total volume of 50 µL, and then subjected to an annealing procedure: initially denaturing at 95°C for 5 minutes, and then cooling from 95°C to 20°C at a rate of 1°C per minute using a thermal cycler. crRNA was synthesized using a T7 high-throughput transcription kit, and the reaction was carried out overnight at 37°C (for about 16 h). RNA was purified using the RNA Clean & Concentrator™-5 (Zymo Research), quantified with NanoDrop 2000C, diluted to a concentration of 10 µM, and stored in a refrigerator at -80°C.
2. PCR amplification: the total volume of the reaction system was 20 µL, 0.25 µM of primers each were used (table 4), 1 ng of a human genome (HEK293T) or directly-scraped oral epithelial mucosa was used as the template, and a high fidelity enzyme KOD FX (ToYoBo) was used for the PCR reaction. The PCR reaction procedure was 95°C for 2 min, and then starting 35 cycles of 98°C for 10 s, 60°C for 15 s, and 68°C for 10 s. After completion of the PCR, the product was directly used for a Casl2a reaction. (Primers 1-rs5082-F-T, 2-rs1467558-F-T, and 3-rs2952768-R-C were directly introduced to the corresponding mutation products of the SNP)
3. Cas12a reaction: a 20 µL reaction system was added with corresponding crRNA (1 µM), LbCas12a (0.5 µM), 1 µL of a PCR product, and a fluorescent probe (HEX-N12-BHQ1) (0.5 µM). Reaction was conducted at 37°C for 15 min, and then terminated at 98°C for 2 min .
4. Fluorescence detection: 20 µL of the inactivated reaction solution was added into a 96-well plate, and then detected by a microplate reader (with an excitation light at 535 nm and an emission light at 556 nm). As shown in FIG. 14, only when the crRNA corresponded to the corresponding target sequence, there would be a higher fluorescence response value, and if there was one point mutation, its response value would be greatly reduced. The genotype of the corresponding SNP could be determined by the fluorescence value, and these results were confirmed by sequencing results.

### Example 8 Testing of a cancer-related gene

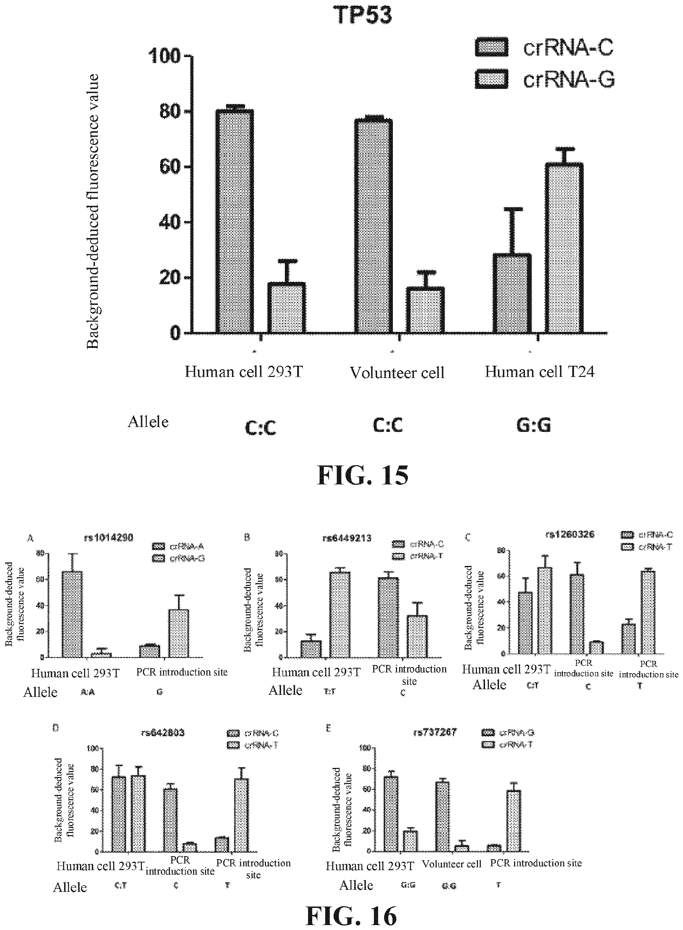

A TP53 gene was selected as the test gene. TP53 gene has a nonsense mutation in a human T24 cell, which leads to inactivation of the gene. A cell with normal gene at this site (HEK293T), an individual gene and a mutant cell T24 were tested respectively.
1. Preparation of crRNA: first, a transcription template was prepared by annealing T7-crRNA-F to synthesized oligonucleotides T7-crRNA-34-TP53-T24-C-16nt and T7-crRNA-34-TP53-T24-G-16nt (Table 5). Specifically, the paired oligonucleotide (4 µM) was annealed in a 1X PCR buffer (Transgen Biotech) at a total volume of 50 µL, and then subjected to an annealing procedure: initially denaturing at 95°C for 5 minutes, and then cooling from 95°C to 20°C at a rate of 1°C per minute using a thermal cycler. crRNA was synthesized using a T7 high-throughput transcription kit, and the reaction was carried out overnight at 37°C (for about 16 h). RNA was purified using the RNA Clean & Concentrator™-5 (Zymo Research), quantified with NanoDrop 2000C, diluted to a concentration of 10 µM, and stored in a refrigerator at -80°C.
2. PCR amplification: the total volume of the reaction system was 20 µL, 0.25 µM of primers 34-TP53-T24-F and 34-TP53-T24-R each were used (Table 4), 1 ng of a human genome (HEK293T, T24) or directly-scraped oral epithelial mucosa was used as the template, and a high fidelity enzyme KOD FX (ToYoBo) was used for the PCR reaction. The PCR reaction procedure was 95°C for 2 min, and then starting 35 cycles of 98°C for 10 s, 60°C for 15 s, and 68°C for 10 s. After completion of the PCR, the product was directly used for a Cas12a reaction.
3. Cas12a reaction: a 20 µL reaction system was added with corresponding crRNA (1 µM), LbCas12a (0.5 µM), 1 µL of a PCR product, and a fluorescent probe (HEX-N12-BHQ1) (0.5 µM). Reaction was conducted at 37°C for 15 min, and then terminated at 98°C for 2 min.
4. Fluorescence detection: 20 µL of the inactivated reaction solution was added into a 96-well plate, and then detected by a microplate reader (with an excitation light at 535 nM and an emission light at 556 nM). As shown in FIG. 15, when the TP53 gene that was normal at this site was the template, the detected value of crRNA-C was significantly higher than that of crRNA-G, while the crRNA-G of the mutant cell T24 was significantly increased.

### Example 9 Testing of Human SNP (Gout-Related Genes)

The SNP test selected 5 sites of human SNP that were related to a gout risk, namely rs1014290, rs6449213, rs737267, rs1260326, and rs642803, to test the HOLMES method.
1. Preparation of crRNA: first, a transcription template was prepared by annealing T7-crRNA-F to a synthesized oligonucleotide (Table 5). Specifically, the paired oligonucleotide (4 µM) was annealed in a 1X PCR buffer (Transgen Biotech) at a total volume of 50 µL, and then subjected to an annealing procedure: initially denaturing at 95°C for 5 minutes, and then cooling from 95°C to 20°C at a rate of 1°C per minute using a thermal cycler. crRNA was synthesized using a T7 high-throughput transcription kit, and the reaction was carried out overnight at 37°C (for about 16 h). RNA was purified using the RNA Clean &ConcentratorTM-5 (Zymo Research), quantified with NanoDrop 2000C, diluted to a concentration of 10 µM, and stored in a refrigerator at -80°C.
2. PCR amplification: the total volume of the reaction system was 20 µL, 0.25 µM of primers each were used (Table 4), 1 ng of a human genome (HEK293T) or directly-scraped oral epithelial mucosa was used as the template, and a high fidelity enzyme KOD FX (ToYoBo) was used for the PCR reaction. The PCR reaction procedure was 95°C for 2 min, and then starting 35 cycles of 98°C for 10 s, 60°C for 15 s, and 68°C for 10 s. After completion of the PCR, the product was directly used for a Casl2a reaction. (Primers 1-rs5082-F-T, 2-rs1467558-F-T, and 3-rs2952768-R-C were directly introduced to the corresponding mutation products of the SNP)
3. Cas12a reaction: a 20 µL reaction system was added with the corresponding crRNA (1 µM), LbCas12a (0.5 µM), 1 µL of a PCR product, and a fluorescence probe (HEX-N12-BHQ1) (0.5 µM). Reaction was conducted at 37°C for 15 min, and then terminated at 98°C for 2 min.
4. Fluorescence detection: 20 µL of the inactivated reaction solution was added into a 96-well plate, and then detected by a microplate reader (with an excitation light at 535 nm and an emission light at 556 nm). As shown in FIG. 16, only when the crRNA corresponded to the corresponding target sequence, there would be a higher fluorescence response value, and if there was one point mutation, its response value would be greatly reduced. The genotype of the corresponding SNP could be determined by the fluorescence value, and these results were confirmed by sequencing results.

### Example 10 SNP Testing of clinical samples of volunteers (a gout related gene) by a kit

A pre-mixed solution was added into a 96-well plate to prepare a kit, and then the kit was added with genomic DNAs of 21 volunteers to test the rs1014290 site, which was related to a gout risk.
1. Preparation of a kit: first, a transcription template was prepared by annealing T7-crRNA-F to a synthesized oligonucleotide (Table 5). Specifically, the paired oligonucleotide (4 µM) was annealed in a 1X PCR buffer (Transgen Biotech) at a total volume of 50 µL, and then subjected to an annealing procedure: initially denaturing at 95°C for 5 minutes, and then cooling from 95°C to 20°C at a rate of 1°C per minute using a thermal cycler. crRNA was synthesized using a T7 high-throughput transcription kit, and the reaction was carried out overnight at 37°C (for about 16 h). RNA was purified using the RNA Clean &ConcentratorTM-5 (Zymo Research), quantified with NanoDrop 2000C, and diluted to a concentration of 10 µM.
2. Pre-mixing in a 96-well plate for PCR: a 19 µL system was added with reagents required for a PCR reaction, the primers being 41-rs1014290-F and 41-rs1014290-R.
3. Pre-mixing in a 96-well plate for fluorescence detection: the 19 µL system was added with a crRNA (1 µM), LbCas12a (0.5 µm) and a fluorescence probe (HEX-N12-BHQ1) (0.5 µM) and was added in the 96-well plate.
4. PCR amplification: the pre-mixed 96-well plate for PCR was added with the genomic DNAs of the volunteers, and then subjected to the PCR reaction. The PCR reaction procedure was 95°C for 2min, and then starting 35 cycles of 98°C for 10 s, 60°C for 15 s, and 68°C for 10 s.
5. Cas12a reaction: 1 µL of a PCR reaction solution was taken and added into the pre-mixed 96-well plate for fluorescence detection, reacted at 37°C for 15 min, and then the reaction was terminated at 98°C for 2 min.
6. Fluorescence detection: it was detected by a microplate reader (with an excitation light at 535 nm and an emission light at 556 nm). As shown in FIG. 17, because a population with a genotype A:A had a higher risk of gout, people other than volunteers Nos. 5, 7 and 9 are of the genotype A:G or G:G, so more attention should be paid to the risk of gout.

### Example 11 Detection of Escherichia coli and the like microorganisms in environmental water by LAMP combined with a Cas protein

The gyrB gene of *Escherichia coli* was selected as the detection target to indirectly test whether *Escherichia coli* or the like microorganisms exist in water.
1. Preparation of crRNA: first, a transcription template was prepared by annealing T7-crRNA-F to a synthesized oligonucleotide T7-crRNA-gyrB (Table 5). Specifically, the paired oligonucleotide (4 µM) was annealed in a 1 X Taq DNA polymerase reaction buffer (Transgen Biotech) at a total volume of 50 µL, and then subjected to an annealing procedure: initially denaturing at 95°C for 5 minutes, and then cooling from 95°C to 20°C at a rate of 1°C per minute using a thermal cycler. crRNA was synthesized using a T7 high-throughput transcription kit, and the reaction was carried out overnight at 37°C (for about 16 h). Then RNA was purified using the RNA purification and concentration kit, quantified with NanoDrop 2000C, finally diluted to a concentration of 10 µM, and stored in a refrigerator at -80°C for later use.
2. LAMP amplification: sterile water and a contaminated liquid containing *Escherichia coli* were used as a negative control and a sample to be detected respectively. The total volume of each reaction system was 25 µL, primers of 1.6 µM of LAMP-FIP and LAMP-BIP each, 0.2 µM of LAMP-F3 and LAMP-B3 each, 0.4 µM of LAMP-LoopF and LAMP-LoopB each were used, and the kit used for the LAMP reaction was a WarmStart® LAMP Kit (NEB). The LAMP reaction procedure was 65°C for 30 min. After the LAMP was completed, annealing was conducted at 80°C for 10 min, and then the product was directly used for a Casl2a reaction.
3. Casl2a reaction: a 20 µL reaction system was added with the crRNA (0.5 µM) purified in step 1, a Casl2a (0.25 µM), 1 µL of a LAMP product, a fluorescence probe (HEX-N12-BHQ1) (0.5 µM), a buffer solution of NEB buffer 3.1, and 0.5 µL of a RNase inhibitor. Reaction was conducted at 37°C for 15 min.
4. Fluorescence detection: 20 µL of the inactivated reaction solution was added into a 96-well plate, and then detected by a microplate reader (with an excitation light at 535 nm and an emission light at 556 nm). The results were as shown in FIG. 19.

### Example 12 Detecting SNP by using LAMP amplification combined with a Cas protein

1. Preparation of crRNA: first, a transcription template was prepared by annealing T7-crRNA-F to a synthesized oligonucleotide T7-crRNA-rs5082-T/T7-crRNA-rs5082-G/T7-crRNA-rs1467558-T/T7-crRNA-rs14 67558-C (Table 5). Specifically, the paired oligonucleotide (4 µM) was annealed in a 1 X Taq DNA polymerase reaction buffer (Transgen Biotech) at a total volume of 50 µL, and then subjected to an annealing procedure: initially denaturing at 95°C for 5 minutes, and then cooling from 95°C to 20°C at a rate of 1°C per minute using a thermal cycler. crRNA was synthesized using a T7 high-throughput transcription kit, and the reaction was carried out overnight at 37°C (for about 16 h). Then RNA was purified using the RNA purification and concentration kit, quantified with NanoDrop 2000C, finally diluted to a concentration of 10 µM, and stored in a refrigerator at -80°C for later use.
2. LAMP amplification: a human genome HEK293T was taken as a sample. The total volume of each reaction system was 25 µL, primers of 1.6 µM of LAMP-FIP and LAMP-BIP each, 0.2 µM of LAMP-F3 and LAMP-B3 each, 0.4 µM of LAMP-LoopF and LAMP-LoopB each were used, and a WarmStart® LAMP Kit (NEB) was used for a LAMP reaction. The LAMP reaction procedure was 65°C for 30 min. After the LAMP was completed, annealing was conducted at 80°C for 10 min, and then the product was directly used for a Cas12a reaction.
3. Cas12a reaction: a 20 µL reaction system was added with the crRNA (0.5 µM) purified in step 1, a Cas12a (0.25 µM), 1 µL of a LAMP product, a fluorescence probe (HEX-N12-BHQ1) (0.5 µM), a buffer solution of NEB buffer 3.1, and 0.5 µL of a RNase inhibitor. Reaction was conducted at 37°C for 15 min.
4. Fluorescence detection: 20 µL of the inactivated reaction solution was added into a 96-well plate, and then detected by a microplate reader (with an excitation light at 535 nm and an emission light at 556 nm). The results were as shown in FIG. 20.

### Example 13 Detection of Escherichia coli and the like microorganisms in environmental water by RPA amplification combined with a Cas protein

The gyrB gene of *Escherichia coli* was selected as the detection target to indirectly test whether *Escherichia coli* and the like microorganisms exist in water.
1. Preparation of crRNA: first, a transcription template was prepared by annealing T7-crRNA-F to a synthesized oligonucleotide T7-crRNA-gyrB (Table 5). Specifically, the paired oligonucleotide (4 µM) was annealed in a 1X PCR buffer (Transgen Biotech) at a total volume of 50 µL, and then subjected to an annealing procedure: initially denaturing at 95°C for 5 minutes, and then cooling from 95°C to 20°C at a rate of 1°C per minute using a thermal cycler. crRNA was synthesized using a T7 high-throughput transcription kit, and the reaction was carried out overnight at 37°C (for about 16 h). Then RNA was purified using the RNA purification and concentration kit, quantified with NanoDrop2000C, finally diluted to a concentration of 10 µM, and stored in a refrigerator at -80°C for later use.
2. RPA amplification: sterile water and a contaminated liquid containing *Escherichia coli* were used as a negative control and a sample to be detected respectively. The total volume of each reaction system was 25 µL, 0.5 µM of primers RPA-gyrB-F (or RPA-gyrB-F2) and RPA-gyrB-R2 each were used, and a TwistAmp® Basic kit (TwistDX) was used for the RPA reaction. The RPA reaction procedure was 37°C for 30 min. After the RPA was completed, annealing was conducted at 80°C for 10 min, and then the product was directly used for a Cas12a reaction.
3. Cas12a reaction: a 20 µL reaction system was added with the crRNA (0.5 µM) purified in step 1, a Cas12a (0.25 µM), 1 µL of a RPA product, a fluorescence probe (HEX-N12-BHQ1) (0.5 µM), a buffer solution of NEB buffer 3.1, and 0.5 µL of a RNase inhibitor. Reaction was conducted at 37°C for 15 min.
4. Fluorescence detection: 20 µL of the inactivated reaction solution was added into a 96-well plate, and then detected by a microplate reader (with an excitation light at 535 nm and an emission light at 556 nm). The results were as shown in FIG. 21.

### Example 14: Cas12b having a collateral cleavage activity

### 1. Preparation of a guide RNA (sgRNA)

Firstly, a plasmid pUC18-guide RNA-T1 was constructed by using pUC18 as the skeleton of the plasmid. In the plasmid, a T7 promoter and a template DNA sequence for transcription of the guide RNA were inserted on the pUC18 (Note: the guide RNA transcribed from this template targeted a sequence called T1 in this study). The method was firstly carrying out a round of PCR by using the pUC18 plasmid as a template and PUC18-1-F and pUC18-1-R as primers; linking PCR products with a T4 DNA Ligase, transforming the product into DH10b, and sequencing to obtain the correct clone, which was called pUC18-guide RNA-T1-pre. Then, a second round of PCR was carried out by using the pUC18-guide RNA-T1-pre as a template and pUC18-2-F and pUC18-2-R as primers, linking and transforming the PCR products in the same way, to finally obtain the plasmid pUC18-guide RNA-T1 which was correct as sequenced.

Next, by using the plasmid pUC18-guide RNA-T1 as a template, a guide RNA was synthesized using a T7 high-throughput transcription kit (Thermo), and the reaction was carried out overnight at 37°C (for 12-16 h).

Finally, the transcription system was added with a DNase I (2 µL of the DNase I was added per 50 µL of the transcription system), subjected to a water bath at 37°C for 30 min to eliminate a plasmid DNA, and the RNA was purified using the RNA purification and concentration kit, quantified with the NanoDrop 2000C, diluted to a concentration of 10 µM, and stored in a refrigerator at -80°C for later use.

### 2. Preparation of a Target DNA

(1) If the target DNA was a single strand, a 66 bp long oligonucleotide was directly synthesized as the target DNA (target-T1-R), which contained the 20 bp target sequence (T1) recognized by the guide RNA.
(2) If the target DNA was double-stranded, two 66 bp long complementary oligonucleotides (target-T1-F; target-T1-R) were directly synthesized, which contained the 20 bp target sequence (T1) identified by the guide RNA. The two oligonucleotides were annealed to obtain a short target DNA. Specifically, the paired oligonucleotide (1 µM) was annealed in a 1X PCR buffer (Transgen Biotech) at a total volume of 20 µL, and then subjected to an annealing procedure: initially denaturing at 95°C for 5 minutes, and then cooling from 95°C to 20°C at a rate of 1°C per minute using a thermal cycler.

### 3. Cas12b reaction

(1) annealing of the guide RNA: the guide RNA was diluted to an appropriate concentration (10 µM), and annealed in the PCR instrument. The annealing procedure: denaturing at 75°C for 5 min, and then cooling from 75°C to 20°C at a decreasing rate of 1°C per minute.
(2) incubation of the guide RNA with C2c1: the annealed guide RNA was mixed with C2c1 at an equal molar concentration, and allowed to stand at 30°C for 20-30 min.
(3) Cas12b reaction: a 20 µL reaction system was added with a mixture of the guide RNA and C2cl incubated in step (2) (the final concentrations of them were both 250 µM or 500 µM), a target DNA (with a final concentration of 50 nM), a FAM-labeled oligonucleotide (target-DNMTI-3-R-FAM-5') or a fluorescence quenching probe (HEX-N12-BHQ1 with the final concentration of 500 nM), 2 µL of a 10 X NEB Buffer 3.1, and 0.5 µL of an RNase inhibitor (40 U/µL). After mixing homogeneously, the reaction was conducted at 48°C for 30 min. After that, it was inactivated by heating at 98°C for 5 min in a PCR instrument.

4. Detection of trans-cleavage activity of Cas12b by urea-denatured gel electrophoresis: 20 µL of the inactivated reaction solution was separated by a urea-denatured gel electrophoresis method, and then imaged by a fluorescence imaging system ImageQuant LAS 4000 mini (GE Healthcare). The results were as shown in FIG. 22.

### 5. Detection of trans-cleavage activity of Cas12b by a fluorescence microplate reader method:

20 µL of the inactivated reaction solution was added into a 96-well plate, and detected by a microplate reader (with an excitation light at 535 nm and an emission light at 556 nm). The resultswere as shown in FIG. 23.

### Example 15: Sensitivity testing of a Cas12b reaction (trans-cleavage)

By detecting the excited fluorescence intensity of the fluorescence probe (HEX-N12-BHQ1), the target DNA concentration required for Cas12b to exert the trans-cleavage activity, i.e., the sensitivity of a Cas12b trans-cleavage reaction, was determined.

### 1. Preparation of a guide RNA

Firstly, by using the pUC18-guide RNA-T1 as a template and guide RNA-DNMT1-3-F and guide RNA-DNMT1-3-R as primers, 20 bases of the guide RNA that target the target DNA of T1 were replaced by a guide RNA targeting DNMT1-3 through PCR, so as to obtain another plasmid pUC 1 8-guide RNA-DNMT1-3.

Then, by using the plasmid pUC18-guide RNA-DNMT1-3 as a template, a guide RNA was synthesized using a T7 high-throughput transcription kit (Thermo), and the reaction was carried out overnight at 37°C (for 12-16 h).

Finally, the transcription system was added with a DNase I (2 µL of the DNase I was added per 50 µL of the transcription system), subjected to a water bath at 37°C for 30min to eliminate a plasmid DNA, and the RNA was purified using the RNA purification and concentration kit, quantified with the NanoDrop 2000C, and stored in a refrigerator at -80°C for later use.

### 2. Preparation of a Target DNA

For the target DNA, the first one is that the target DNA was directly added into a Cas12b reaction system without amplification. The method was as follows:
(1) If the target DNA was a single strand, a 50 bp long oligonucleotide was directly synthesized as the target DNA (DNMT1-3 (TTC PAM)-R), which contained the 20 bp target sequence (DNMT1-3) recognized by the guide RNA.
(2) If the target DNA was double-stranded, two 50 bp long complementary oligonucleotides (DNMT1-3 (TTC PAM)-F; DNMT1-3 (TTC PAM)-R) were directly synthesized, which contained the 20 bp target sequence (DNMT1-3) recognized by the guide RNA. The two oligonucleotides were annealed to obtain a short target DNA. Specifically, the paired oligonucleotide (2 µM) was annealed in a 1X PCR buffer (Transgen Biotech) at a total volume of 20 µL, and then subjected to an annealing procedure: denaturing at 95°C for 5 minutes, and then cooling from 95°C to 20°C at a rate of 1°C per minute using a thermal cycler.
(3) the single-stranded or double-stranded target DNA was diluted at a gradient to 2 µM, 0.2 µM, 0.02 µM, 0.002 µM and 0.0002 µM for later use.

The second one was inserting a fragment containing the target sequence (DNMT1-3) into a plasmid vector for amplification by the LAMP reaction.
(1) the fragment containing the target sequence (DNMT1-3) was inserted into a pEasy-Blunt Zero Cloning Vector using a pEasy-Blunt Zero Cloning Kit from Transgen, so as to obtain the correct clone after verified by sequencing.
(2) A LAMP expansion reaction

Using the above plasmid as a template, the LAMP amplification reaction was carried out. The templates were added respectively at 0 nM, 1 nM, and 0.1 nM, and diluted at a 10-fold gradient to 10⁻¹¹ nM. The total volume of each reaction system was 25 µL, primers of 1.6 µM of LAMP-DNM-FIP and LAMP-DNM-BIP each, 0.2 µM of LAMP-DNM-F3 and LAMP-DNM-B3 each, 0.4 µM of LAMP-DNM-LoopF and LAMP-DNM-LoopB each were used, and the kit used for the LAMP reaction was the WarmStart® LAMP Kit (NEB). The LAMP reaction procedure was 65°C for 30 min. After the LAMP was completed, inactivation was conducted at 80°C for 10 min, and then the product was directly used for a Cas12b reaction.

### 3. Cas12b reaction

(1) annealing of the guide RNA: the guide RNA was diluted to an appropriate concentration (5 µM), and annealed in the PCR instrument. The annealing procedure: denaturing at 75°C for 5 min, and then cooling from 75°C to 20°C at a decreasing rate of 1°C per minute.
(2) incubation of the guide RNA with Cas12b : the annealed guide RNA was mixed with Cas12b at an equal molar concentration, and allowed to stand at 30°C for 20-30 min.
(3) Cas12b reaction: a 20 µL reaction system was added with the mixture of the guide RNA and Cas12b incubated in step (2) (the final concentrations of the guide RNA and Cas12b were both 250 µM), 1 µL of a target DNA or 1 µL of a LAMP product, a fluorescence probe (HEX-N12-BHQ1) (with a final concentration of 500 nM), 2 µL of a 10X NEB Buffer 3.1, and 0.5 µL of an RNase inhibitor (40 U/µL). After mixing homogenously, the reaction was conducted at 48°C for 30min. After that, it was inactivated fire heating at 98°C for 5 min in a PCR instrument.

### 4. Detection of trans-cleavage activity of Cas12b by a fluorescence microplate reader method:

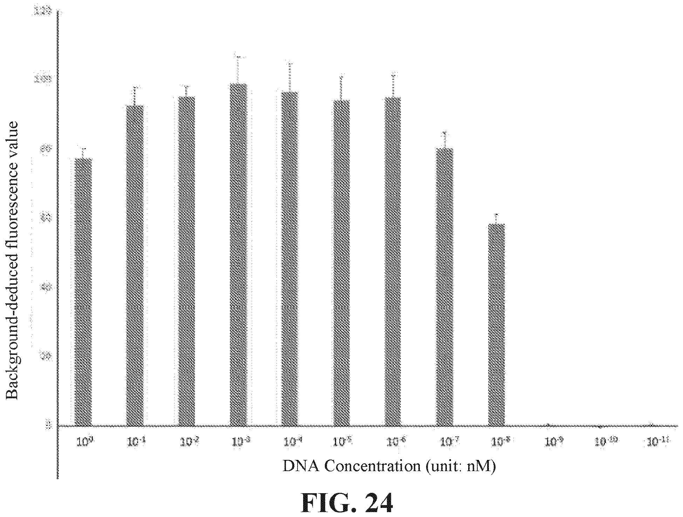

20 µL of the inactivated reaction solution was added into a 96-well plate, and detected by a microplate reader (with an excitation light at 535 nm and an emission light at 556 nm). Combined with the LAMP amplification, Cas12b could produce a significant collateral single-stranded DNA trans-cleavage activity for a target DNA concentration as low as 10 aM. The results were as shown in FIG. 24.

### Cis-cleavage characteristics of Cas12a in cleaving a target single-stranded DNA:

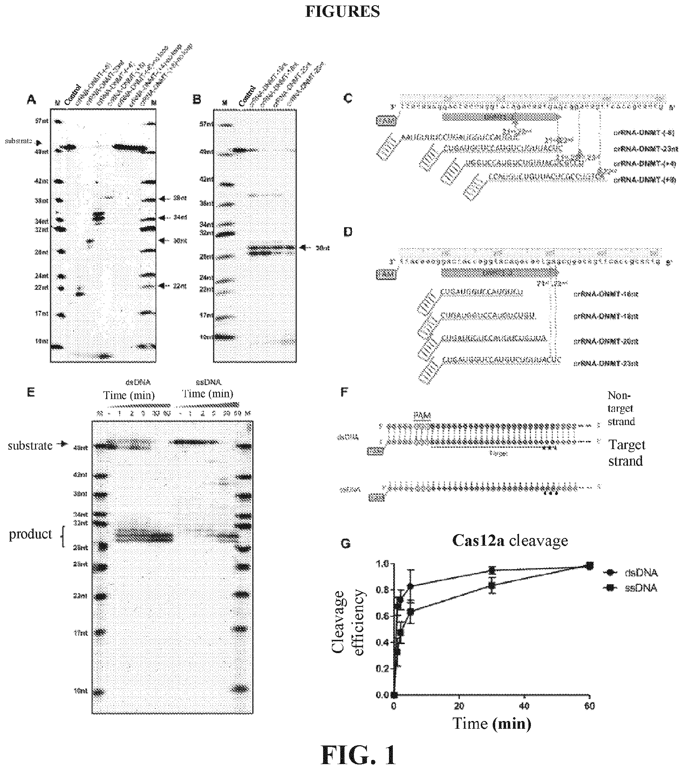

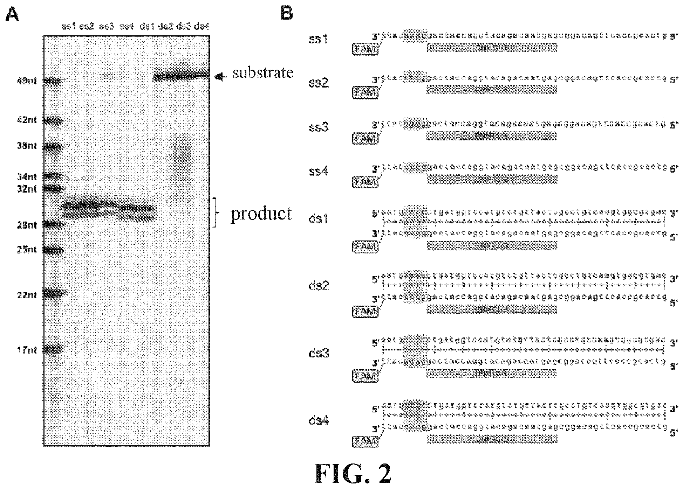

First, in order to test the single-stranded DNA cleavage characteristics of Cas12a, several crRNAs targeting a short single-stranded DNA (DNMT1-3) (Table 1) are designed, which are labeled with 5(6)-carboxyfluorescein (FAM) at the 3' terminal. After the cleavage by FnCasl2a, the reaction product is analyzed by denatured urea-polyacrylamide gel electrophoresis (urea-PAGE). It is found that the single strand DNA cleavage by Cas12a is programmed. That is, the cleavage site is near the 22nd base (21st to 23rd bases) of the target sequence counted from the 3' end base to the 5' end of the first target sequence paired with the crRNA guide sequence, as shown in FIGs. 1A and 1C. The cleavage of a double-stranded DNA by Cas12a requires a PAM sequence, while the cleavage of a single-stranded DNA by Cas12a does not require the PAM sequence (FIGs. 1A, 1B and 2), which is similar to the single-stranded DNA cleavage mediated by Cas9. However, the single-stranded DNA cleavage activity mediated by Casl2a depends on a stem-loop structure in crRNA, as shown in FIG. 1A, while Cas9 still shows weak cleavage activity for a single-stranded DNA with only a 20-nt complementary RNA sequence. The stem-loop structure of the crRNA is important for stabilizing the structure of Cas12a, which is the reason why the ring structure of the crRNA is necessary for the cleavage of the single-stranded DNA by Cas12a. It is further tested whether a shorter lead sequence crRNA can pass through the single-stranded DNA cleavage site of Cas12a in such a manner that the cleavage is outside the recognition site. When the length of the guide sequence is 16 nt, 18 nt and 20 nt, all of these crRNAs lead to cleavage by Cpf1 near the 22nd base, as shown in FIGs. 1B and 1D, meaning that the cleavage site is 4 nt, 2 nt or 0 nt outside the recognition site. Next, the cleavage efficiency of Cas12a on different substrates is tested by using substrates of a double-stranded DNA and a single-stranded DNA respectively, as shown in FIG. 1F. Similar to the situation of Cas9 cleavage, the cleavage of the single-stranded DNA is slower than that of the double-stranded DNA, as shown in FIGs. 1E to 1G. These results indicate that the mechanism of recognizing and cleaving a single-stranded DNA by Cas12a may be different from that of a double-stranded DNA, which is a low-efficiency recognition and cleavage manner independent of PAM; and the PAM sequence accelerates the recognition and/or cleavage of the target double-stranded DNA by Cas12a.

### Trans-cleavage characteristics of Cas12a in cleaving a single-stranded DNA:

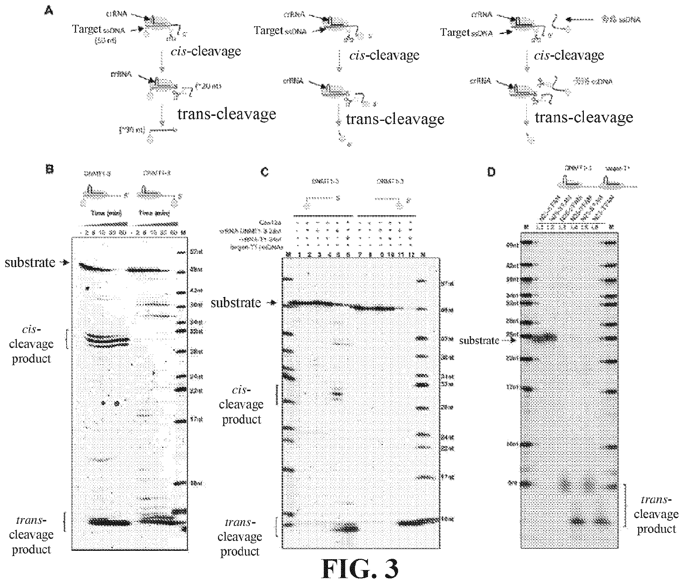

When the target single-stranded DNA is labeled at the 3' terminal, Cas12a cleaves at the vicinity of the 22nd base, as shown in FIG. 1. However, when it is labeled at the 5' terminal, no cleavage product band of predicted size is observed, but a short (< 6 nt) FAM-labeled product is produced, as shown in FIG. 3B. Through detailed experiments, once the ternary complex Cas12a/crRNA/target single-stranded DNA is formed, the target single-stranded DNA (DNMT1-3) (Table 1) labeled at 5'-terminal is cleaved, and a short FAM-labeled product is produced, as shown in FIG. 3C. In addition, the ternary complex also cleaves a single-stranded DNA that has no sequence that is complementary with the crRNA (i.e., a collateral single-stranded DNA) in any other reaction system, as shown in FIGs. 3C and 3D. This cleavage phenomenon is called trans-cleavage, which is different from programmable cis-cleavage. When the target single-stranded DNA is labeled at the 3' terminal, trans-cleavage is also observed, but many cis-cleavage products are left, as shown in FIG. 3B, This may be due to the complex formed by Cas12a/crRNA/target single-stranded DNA, and the target single-stranded DNA is protected, such that its labeled 3' terminal is protected from exposure to a nuclease active site of the ternary complex. These cleavage processes can be as shown in FIG. 3A.

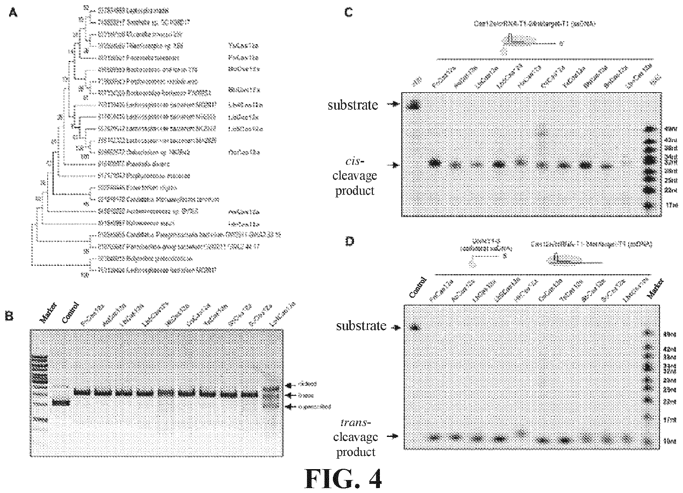

In addition to the FnCas12a tested above, 9 Cas12as from other species sources are also tested (Table 2 and FIG. 4A). Except Lb4Cas12a, all Cas12as have good endonuclease activity to the plasmid DNA (as shown in FIG. 4B), and all Cas12a ternary complexes show cis and trans cleavage activities on the single strand (as shown in FIGs. 4C and 4D). This shows that cis and trans activity of Cas12a on the single-stranded DNA is a common phenomenon.

### The cis and trans key sites and mechanism for cleaving a single-stranded DNA by Cas12a.

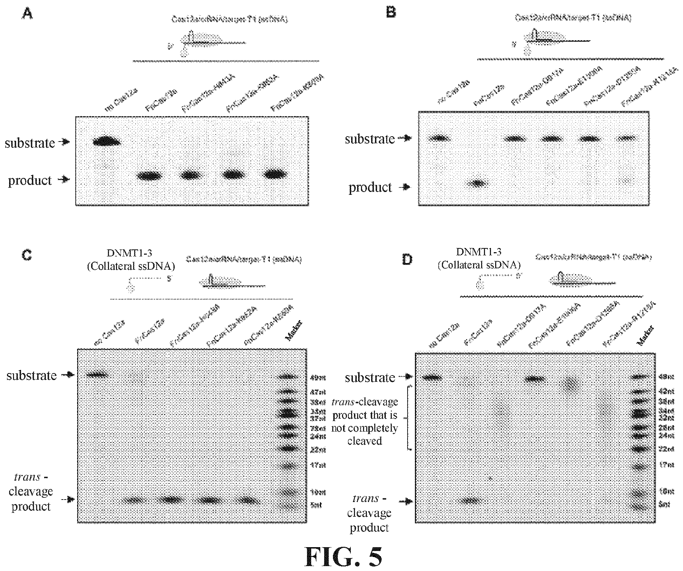

In order to determine the key amino acid residues related to cis and trans activities on the single-stranded DNA in Cas12a, several candidate residues of Cas12a are mutated to conduct the activity test. First, three single-amino-acid mutants of FnCas12a (H843A, K852A and K869A) are purified and tested, and their residues are related to an RNase activity. The results of the study of the trans-activity on the single-stranded DNA show that no obvious difference in cis- and trans-cleavage activities on the single-stranded DNA between the wild-type FnCas12a and the three mutants is found, as shown in FIGs. 5A and 5C.

Next, when endonuclease active sites in the FnCas12a, i.e., the RuvC domain (D917A, E1006A or D1255A) and the Nuc domain (R1218A) sites are mutated, the cis and trans cleavage activities of these mutated Cas12a on the single-stranded DNA are both affected, as shown in FIGs. 5B and 5D. These results indicate that the key site of Cas12a for cleavage of a target double-stranded DNA is closely related to cis and trans cleavage activities on the single-stranded DNA.

Recent structural studies of Cas12b (i.e., C2c1) (including complexes with an extended target DNA or an extended non-target DNA) show that both strands are located in an RuvC pocket, as shown in FIGs. 6A and 6B. By comparing the endonuclease catalytic residues of Cas12b (i.e. C2c1) and Cas12a, these sites are most likely to play similar roles in the cleavage and functions of Cas12b (i.e., C2c1) and Casl2a. The results of an in vitro single amino acid mutation experiment show that it is consistent with the above hypothesis. That is, Casl2a is likely to cleave two strands through only one RuvC catalytic pocket.

The trans-cleavage activity of a Cas12a complex: in the structure of a complex of Cas12b (i.e., C2c1) with an additional single-stranded DNA, a sequence-independent single-stranded DNA is also located on the surface of a catalytic pocket, as shown in FIG. 6C, which is similar to that of a collateral single-stranded DNA substrate in Cas12a. Combined with a single-amino-acid mutation experiment, it is proposed that the target DNA, non-target DNA and collateral single-stranded DNA are all cleaved in the single RuvC pocket in Cas12a, as shown in FIGs. 6D, 6E and 6F. The ternary Cas12a complex has a collateral single-stranded DNA trans-cleavage activity, while the reason why a monomer or a binary complex does not have the collateral single-stranded DNA trans-cleavage activity can be explained by comparing the structures of the monomer, binary and ternary complexes. The structure of a monomer Cas12a is disordered, the binary complex Cas12a/crRNA has a triangular structure, as shown in FIG. 6G, while the ternary complex Cas12a/crRNA/target DNA is converted into a double-leaf structure, thus exposing the catalytic pocket to realize trans-cleavage of the collateral single-stranded DNA (as shown in FIG. 6H).

### Establishment of a nucleic acid detection method

Based on the characteristics of Cas12a, a specific nucleic acid molecule detection method was developed, which was called HOLMES (one HOur Low-cost Multipurpose Efficient Simple assay). Just like the name of the technology, it was characterized by a simple test method that is one hour and has low cost, multiple uses and high efficiency.

In the whole reaction system, the method can be divided into two major steps, one is the amplification of a template nucleic acid, and the other is the specific nucleic acid detection by a Cas12a protein. Here, a PCR method is used for nucleic acid amplification, but in fact, any amplification method can be combined with the nucleic acid detection in the second step, such as the isothermal amplification method RPA, etc. The initial nucleic acid is not limited to a double-stranded DNA, but may also be a single-stranded DNA; or even an RNA still can be detected after reverse transcription, so this method is suitable for various types of nucleic acid molecules. For the nucleic acid detection phase, three components are the keys to the experiment, namely Cas12a, crRNA and a nucleic acid probe. In addition to the 10 Cas12as mentioned in the example (these 10 proteins are randomly selected), other Cas12a proteins are also suitable for this method. In addition, other types of Cas proteins (e.g., a C2c1 protein) are also suitable for the claimed scope of the present invention: as shown by the experimental results, Alicyclobacillus acidoterrestris Cas12b (i.e., C2c1) also has a collateral single-stranded DNA trans-cleavage activity similar to that of Cas12a, and its complex with crRNA/target DNA can also cleave a collateral singlestranded DNA.

For crRNA serving as a guide, it will be more stable in the system after being engineered, for example being modified manually. With regard to the selection of nucleic acid probes, the present invention selects a short single-stranded DNA labeled with HEX and BHQ1, and any other detectable labeling method is theoretically applicable, as long as the nucleic acid probe is cleaved to produce detectable differences. Alternatively, the nucleic acid probe can also be designed to fluoresce after binding to a compound, so as to detect whether the probe is cleaved.

Furthermore, it should be understood that after reading the above teachings of the present invention, those skilled in the art can make various changes or modifications to the present invention, and these equivalent forms also fall within the scope defined by the appended claims of the present application.

**Table 1 Substrate sequences of Cas12a characteristic experiment-related cleavage**

| **Oligo Name** | **Sequence (5'-3')** | **SEQ ID No. :** |
|---|---|---|
| target-DMT1-3-F | aatgtttcctgatggtccatgtctgttactcgc ctgtcaagtggcgtgac | 1 |
| target-DNMT1-3-R | gtcacgccacttgacaggcgagtaacagacatg gaccatcaggaaacatt | 2 |
| target-DNMT1-3-R-FAM-3' | gtcacgccacttgacaggcgagtaacagacatg gaccatcaggaaacatt-FAM | 3 |
| target-DNMT1-S-R-FAM-5' | FAM-gtcacgccacttgacaggcgagtaacaga catggaccatcaggaaacatt | 4 |
| target-T1-F | tttctgtttgttatcgcaactttctactgaatt caagctttactctagaaagaggagaaaggatcc | 5 |
| target-T1-R | ggatcctttctcctctttctagagtaaagcttg aattcagtagaaagttgcgataacaaacagaaa | 6 |
| target-T1-F-FAM | FAM-tttctgtttgttatcgcaactttctactg aattcaagctttactctagaaagaggagaaagg atcc | 7 |
| target-T1-R-FAM | : ggatcctttctcctctttctagagtaaagcttg aattcagtagaaagttgcgataacaaacagaaa -FAM | 8 |
| target-T1-FAM-3' -F | tttctgtttgttatcgcaactttctactgaatt caagctttactctagaaagaggagaaaggatcc -FAM | 9 |
| target-T1-FAM-5' -R | FAM-ggatcctttctcctctttctagagtaaag cttgaattcagtagaaagttgcgataacaaaca | 10 |
| | gaaa | |
| target-DNMT1-3-R-TTT-FAM-3' | gtcacgccacttgacaggcgagtaacagacatg gaccatcaggTTTcatt-FAM | 11 |
| target-DNMT1-3-R-CCC-FAM-3' | gtcacgccacttgacaggcgagtaacagacatg gaccatcaggCCCcatt-FAM | 12 |
| ta rge t-DNMT1-3-R-GGG-FAM-3' | gtcacgccacttgacaggcgagtaacagacatg gaccatcaggGGGcatt-FAM | 13 |
| target-DNMT1-3-F-AAA | aatgAAAcctgatggtccatgtctgttactcgc ctgtcaagtggcgtgac | 14 |
| target-DNMT1-3-F-GGG | aatgGGGcctgatggtccatgtctgttactcgc ctgtcaagtggcgtgac | 15 |
| target-DNMT1-3-F-CCC | aatgCCCcctgatggtccatgtctgttactcgc ctgtcaagtggcgtgac | 16 |
| target-T1-1-R | acaaacagaaa | 17 |
| target-T1-6-R | cgataacaaacagaaa | 18 |
| Larget-T1-12-R | aagttgcgataacaaacagaaa | 19 |
| target-T1-18-R | agtagaaagttgcgataacaaacagaaa | 20 |
| target-T1-24-R | gaattcagtagaaagttgcgataacaaacagaa a | 21 |
| target-T1-24-only-R | gaattcagtagaaagttgcgataa | 22 |
| target-T1-18-only-R | agtagaaagttgcgataa | 23 |
| target-T1-12-only-R | aagttgcgataa | 24 |
| target-T1-6-only-R | cgataa | 25 |
| N25-5' F AM | FAM-NNNNNNNNNNNNNNNNNNNNNNNNN | 26 |
| N25-3' FAM | NNNNNNNNNNNNNNNNNNNNNNNNN-FAM | 27 |

**Table 2. Names and GI numbers of the Casl2a protein and the Cas12b (i.e., C2cl) protein**

| **Name** | **GI number** | **Species** |
|---|---|---|
| FnCas12a | 489130501 | Francisella tularensis |
| AsCas12a | 545612232 | Acidaminococcus sp. BV3L6 |
| LbCas12a | 917059416 | Lachnospiraceae bacterium ND2006 |
| Lb5Cas12a | 652820612 | Lachnospiraceae bacterium NC2008 |
| HkCas12a | 491540987 | Helcococcus kunzii ATCC 51366 |
| OsCas12a | 909652572 | Oribacterium sp. NK2B42 |
| TsCas12a | 972924080 | Thiomicrospira sp. XS5 |
| BbCas12a | 987324269 | Bacteroidales bacterium KAD0251 |
| BoCas12a | 496509559 | Bacteroidetes oral taxon 274 str. F0058 |
| Lb4Cas12a | 769130406 | Lachnospiraceae bacterium MC2017 |
| C2c1 | 1076761101 | Alicyclobacillus acidoterrestris |

**Table 3 Plasmid Information**

| Plasmids or Strains | Relevant properties or genotypes | Sources |
|---|---|---|
| Plasmids | | |
| pET28a-TEV | pET28a with the thrombin cleavage site changed to the TEV protease cleavage site | (Carneiro, Silva et al. 2006) |
| pET28a-TEV-FnCas12a | pET28a-TEV carrying FnCas12a | (Li, Zhao et al. 2016) |
| pET28a-TEV-AsCas12a | pET28a-TEV carrying AsCas12a | (Li, Zhao et. al. 2016) |
| pET28a-TEV-LbCas12a | pET28a-TEV carrying LbCas12a | (Lei, Li et al. 2017) |
| pET28a-TEV-Lb5Cas12a | pET28a-TEV carrying Lb5Cas12a | The present invention |
| pET28a-TEV-HkCas12a | pET28a-TEV carrying HkCas12a | The present invention |
| pET28a-TEV-0sCas12a | pET28a-TEV carrying 0sCas12a | The present invention |
| pET28a-TEV-TsCas12a | pET28a-TEV carrying TsCas12a | The present invention |
| pET28a-TEV-BbCas12a | pET28a-TEV carrying BbCas12a | The present invention |
| pET28a-TEV-BoCas12a | pET28a-TEV carrying BoCas12a | The present invention |
| pET28a-TEV-Lb4Cas12a | pET28a-TEV carrying Lb4Cas12a | The present invention |
| pET28a-TEV-FnCas12a-K869A | pET28a-TEV carrying FnCas12a-K869A | The present invention |
| pET28a-TEV-FnCas12a-K852A | pET28a-TEV carrying FnCas12a-K852A | The present invention |
| pET28a-TEV-FnCas12a-H843A | pET28a-TEV carrying FnCas12a-H843A | The present invention |
| pET28a-TEV-FnCas12a-R1218A | pET28a-TEV carrying FnCas12a-R1218A | The present invention |
| pET28a-TEV-FnCas12a-E1006A | pET28a-TEV carrying FnCas12a-E1006A | The present invention |
| pET28a-TEV-FnCas12a-D917A | pET28a-TEV carrying FnCas12a-D917A | The present invention |
| pET28a-TEV-FnCas12a-D1255A | pET28a-TEV carrying FnCas12a-D1255A | The present invention |
| pET28a-TEV-C2c1 | pET28a-TEV carrying C2c1 | The present invention |

**Table 4 Primers used in the test of the HOLMES method**

| **Oligo Name** | **Sequence (5'-3')** | **SEQ ID No. :** |
|---|---|---|
| target-T1-R | ggatcctttctcctctttctagagtaaagcttgaa ttcagtagaaagttgcgataacaaacagaaa | 28 |
| M13F-47 | cacaattccacacaacatacgagccgga | 29 |
| M13R-48 | tgtagccgtagttaggccaccacttca | 30 |
| Target-T1-F | agttttgttatcgcaactttctactgaattc | 31 |
| Target-T1-F-1A | agttttgAtatcgcaactttctactgaattc | 32 |
| Target-T1-F-2A | agttttgtAatcgcaactttctactgaattc | 33 |
| Target-T1-F-3T | agttttgttTtcgcaactttctactgaattc | 34 |
| Target-T1-F-4A | agttttgttaAcgcaactttctactgaattc | 35 |
| Target-T1-F-5G | agttttgttatGgcaactttctactgaattc | 36 |
| Target-T1-F-6C | agttttgttatcCcaactttctactgaattc | 37 |
| Target-T1-F-7G | agttttgttatcgGaactttctactgaattc | 38 |
| Target-T1-F-8T | agttttgttatcgcTactttctactgaattc | 39 |
| Target-T1-F-9T | agttttgttatcgcaTctttctactgaattc | 40 |
| Target-T1-F-10G | agttttgttatcgcaaGtttctactgaattc | 41 |
| Target-T1-AAAN-F | aaaagttatcgcaactttctactgaattc | 42 |
| Target-T1-F-11A | agttttgttatcgcaacAttctactgaattcggtc atag | 43 |
| Target-T1-F-12A | agttttgttatcgcaactAtctactgaattcggtc atag | 44 |
| Target-T1-F-13A | agttttgttatcgcaacttActactgaattcggtc atag | 45 |
| Target-T1-F-14G | agttttgttatcgcaactttGtactgaattcggtc atag | 46 |
| Target-T1-F-15A | agttttgttatcgcaactttcAactgaattcggtc atag | 47 |
| Target-T1-F-16T | agttttgttatcgcaactttctTctgaattcggtc atag | 48 |
| Target-T1-F-17G | agttttgttatcgcaactttctaGtgaattcggtc atag | 49 |
| Target-T1-F-18A | agttttgttatcgcaactttctacAgaattcggtc atag | 50 |
| Target-T1-PAMIA-F | agtttAgttatcgcaactttctactgaattc | 51 |
| Target-T1-PAM2A-F | agttAtgttatcgcaactttctactgaattc | 52 |
| Target-T1-PAM3A-F | agtAttgttatcgcaactttctactgaattc | 53 |
| gyrB-F | AGTTGTCGTTCCTCAACTCCGGCGTTTC | 54 |
| gyrB-R | TCGACGCCAATACCGTCTTTTTCAGTGG | 55 |
| 1-5082-F | CTGCCTTTGCTTCTACCTTTGCCTGT | 56 |
| 1-5082-F-T | TTGCTTCTACCTTTGCCTGTTCTGG | 57 |
| 1-5082-R | TTTTCTGGCTGGGGATGGCCGATGG | 58 |
| 2-rs1467558-F | AGCAATAACACTAATATTGATTCCTTCAGATATGG ACTCCTTTCATAGTA | 59 |
| 2-rs1467558-F-T | TTGATTCCTTCAGATATGGACTCCTTTCATAGTAT AACG | 60 |
| 2-rs1467558-R | TGAGCATCGTTATTCTTACGCGTTGTCATTGAAAG AG | 61 |
| 3-rs2952768-F | AGCCTGGGCAACGAGTGAAACTCTG | 62 |
| 3-rs2952768-R | ACAGGAGGGACAAAGGCCTAAGTGTCC | 63 |
| 3-rs2952768-R-C | CATCATAGGATTGGGAAAAGGACATTTCAGTCATT CAG | 64 |
| 4-rs4363657-F | AGAGTCCTTCTTTCTCAATTTTTCAGAATAATTTA GTACTTTGGGTAC | 65 |
| 4-rs4363657-R | CAGTACTGAAAAAACCTGCCTATCAATAAAAGCCC TAGAC | 66 |
| 5-rs601338-F | GCTTCACCGGCTACCTTTGCTCCT | 67 |
| 5-rs601338-R | TTCACCTGCAGGCCCCCGCAGG | 68 |
| 34-TP53-T24-F | CCTGACTTTCAACTCTGTCTCCTTCCTCTTTTTAC AGTA | 69 |
| 34-TP53-T24-R | TGCTGTGACTGCTTGTAGATGGCCATGG | 70 |
| 41-rs1014290-F | AGTTTCCAGACCTCAGTGCACAAGATACTTTTCTA C | 71 |
| 41-rs1014290-F-G | ACCTCAGTGCACAAGATACTTTTCTACGTCATCCA C | 72 |
| 41-rs1014290-R | AGCTCCAGTGGATGGAAGATCTTTGAGATCCAG | 73 |
| 42-rs6449213-F | AGTCAAAGAGATTCATGCCTGGGACTTTAATCACA TTTAT | 74 |
| 42-rs6449213-F-C | ATGCCTGGGACTTTAATCACATTTATCGGAAGG | 75 |
| 42-rs6449213-R | CAAATCTGTCTCCACCTCTCAGCTCACCTTG | 76 |
| 43-rs737267-F | TTCTTGAACCCAAACTCACCTGGCATTTAAACTG | 77 |
| 43-rs737267-F-A | AAACTCACCTGCCATTTAAACTGTCTAAG | 78 |
| 43-rs737267-F-T | AAACTCACCTGGCATTTAAACTGTCTCTGTAAG | 79 |
| 43-rs737267-R | TGCCGAGGCTGAGTTCAGCTACTCTCC | 80 |
| 44-rs1260326-F | ACACAGCACCGTGGGTCAGACCTTGC | 81 |
| 44-rs1260326-F-C | TGGGTCAGACTTTGCCGGTGAGAGTC | 82 |
| 44-rs1260326-F-T | TGGGTCAGACTTTGCTGGTGAGAGTC | 83 |
| 44-rs1260326-R | AGCAGTGGCCATGTGATGCTGATGATG | 84 |
| 45-rs642803-F | CCCCGGCTCTGTTGGCTTTGAGAATTG | 85 |
| 45-rs642803-F-C | CTCTGTTGGCTTTGAGAATTGCCTGTCTGTGTC | 86 |
| 45-rs642803-F-T | CTCTGTTGGCTTTGAGAATTGTCTGTCTGTGTC | 87 |
| 45-rs642803-R | ACCGATACCTGGCAGCCCTTGGATG | 88 |
| HEX-N12-BHQ1 | HEX-NNNNNNNNNNNN-BHQ1 | 89 |

**Table 5 Template sequences for transcription of crRNA**

| **Oligo Name** | **Sequence (5'-3')** | **SEQ ID No. :** |
|---|---|---|
| T7-crRNA-F | GAAATTAATACGACTCACTATAGGG | 90 |
| | gaattcagtagaaagttgcgataaATCTACAACAGTAGA AATTCCCTATAGTGAGTCGTATTAATTTC | 91 |
| T7-T1-15-R | agaaagttgcgataaATCTACAACAGTAGAAATTCCCTA TAGTGAGTCGTATTAATTTC | 92 |
| T7-T1-16-R | tagaaagttgcgataaATCTACAACAGTAGAAATTCCCT ATAGTGAGTCGTATTAATTTC | 93 |
| T7-T1-17-R | gtagaaagttgcgataaATCTACAACAGTAGAAATTCCC TATAGTGAGTCGTATTAATTTC | 94 |
| T7-T1-18-R | agtagaaagttgcgataaATCTACAACAGTAGAAATTCC CTATAGTGAGTCGTATTAATTTC | 95 |
| T7-crRNA-DNMT-23nt-R | GAGTAACAGACATGGACCATCAGATCTACAACAGTAGAA ATTCCCTATAGTGAGTCGTATTAATTTC | 96 |
| T7-crRNA-DNMT-(-8)-R | gacatggaccatcaggaaacattATCTACAACAGTAGAA ATTCCCTATAGTGAGTCGTATTAATTTC | 97 |
| T7-crRNA-DNMT-(+4)-R | aggcgagtaacagacatggaccaATCTACAACAGTAGAA ATTCCCTATAGTGAGTCGTATTAATTTC | 98 |
| T7-crRNA-DNMT-(+8)-R | tgacaggcgagtaacagacatggATCTACAACAGTAGAA | 99 |
| | ATTCCCTATAGTGAGTCGTATTAATTTC | |
| T7-crRNA-DNMT-16nt-R | agacatggaccatcagATCTACAACAGTAGAAATTCCCT ATAGTGAGTCGTATTAATTTC | 100 |
| T7-crRNA-DNMT-18nt-R | acagacatggaccatcagATCTACAACAGTAGAAATTCC CTATAGTGAGTCGTATTAATTTC | 101 |
| T7-crRNA-DNMT-20nt-R | taacagacatggaccatcagATCTACAACAGTAGAAATT CCCTATAGTGAGTCGTATTAATTTC | 102 |
| T7-DNMT-(-8)-no loop-R | gacatggaccatcaggaaacattCCCTATAGTGAGTCGT ATTAATTTC | 103 |
| T7-DNMT-(+4)-no loop-R | aggcgagtaacagacatggaccaCCCTATAGTGAGTCGT ATTAATTTC | 104 |
| T7-DNMT-(+8)-no loop-R | tgacaggcgagtaacagacatggCCCTATAGTGAGTCGT ATTAATTTC | 105 |
| T7-crRNA-rs5082-T | CCTCTTCCCAGAACAGGATCTACAACAGTAGAAATTCCCT ATAGTGAGTCGTATTAATTTC | 106 |
| T7-crRNA-rs5032-G | CCTCTTCCCAGCACAGGATCTACAACAGTAGAAATTCCCT ATAGTGAGTCGTATTAATTTC | 107 |
| F7-crRNA-rs1467558-T | CTGAAGCGTTATACTATATCTACAACAGTAGAAATTCCCT ATAGTGAGTCGTATTAATTTC | 108 |
| T7-crRNA-rs1467558-C | CTGAAGCGTTGTACTATATCTACAACAGTAGAAATTCCCT ATAGTGAGTCGTATTAATTTC | 109 |
| T7-crRNA-rs2952768-T-16nt | TTTTATCTGAATGATTATCTACAACAGTAGAAATTCCCTA TAGTGAGTCGTATTAATTTC | 110 |
| T7-crRNA-rs2952768-C-16nt | TTTTATCTGAATGACTATCTACAACAGTAGAAATTCCCTA TAGTGAGTCGTATTAATTTC | 111 |
| T7-crRNA-rs4363657-T | AAAAAAGAGTGAGTACCATCTACAACAGTAGAAATTCCCT ATAGTGAGTCGTATTAATTTC | 112 |
| r7-crRNA-rs4363657-C | AAAAAAGAGTGGGTACCATCTACAACAGTAGAAATTCCCT ATAGTGAGTCGTATTAATTTC | 113 |
| T7-crRNA-rs601338-G | GGTAGAAGGTCCAGGAGATCTACAACAGTAGAAATTCCCT | 114 |
| | ATAGTGAGTCGTATTAATTTC | |
| T7-crRNA-rs601338-A | GGTAGAAGGTCTAGGAGATCTACAACAGTAGAAATTCCCT ATAGTGAGTCGTATTAATTTC | 115 |
| T7-crRNA-34-TP53-T24-C-16nt | GGGCAGGGGAGTACTGATCTACAACAGTAGAAATTCCCTA TAGTGAGTCGTATTAATTTC | 116 |
| T7-crRNA-34-TP53-T24-G-16nt | GGGCAGGGGACTACTGATCTACAACAGTAGAAATTCCCTA TAGTGAGTCGTATTAATTTC | 117 |
| T7-crRNA-41-rs1014290-A-15nt | TCAGTGGATGATGTAATCTACAACAGTAGAAATTCCCTAT AGTGAGTCGTATTAATTTC | 118 |
| T7-crRNA-41-rs10142901-G-15nt | TCAGTGGATGACGTAATCTACAACAGTAGAAATTCCCTAT AGTGAGTCGTATTAATTTC | 119 |
| T7-crRNA-42-rs6449213-C | GGAAATTCTCCTTCCGAATCTACAACAGTAGAAATTCCCT ATAGTGAGTCGTATTAATTTC | 120 |
| T7-crRNA-42-rs6449213-T | GGAAATTCTCCTTCCAAATCTACAACAGTAGAAATTCCCT ATAGTGAGTCGTATTAATTTC | 121 |
| T7-crRNA-43-rs73T267-A-16nt | TCTTACAGAGTCAGTTATCTACAACAGTAGAAATTCCCTA TAGTGAGTCGTATTAATTTC | 122 |
| T7-crRNA-43-rs737267-G-16nt | TCTTACAGAGCCAGTTATCTACAACAGTAGAAATTCCCTA TAGTGAGTCGTATTAATTTC | 123 |
| T7-crRNA-43-rs737267-T | 3TCTTACAGAGACAGTTATCTACAACAGTAGAAATTCCCT ATAGTGAGTCGTATTAATTTC | 124 |
| T7-crRNA-44-r-s1260326-C-15nt | CTGGACTCTCACCGGATCTACAACAGTAGAAATTCCCTAT AGTGAGTCGTATTAATTTC | 125 |
| T7-crRNA-44-rs1260326-T-15nt | CTGGACTCTCACCAGATCTACAACAGTAGAAATTCCCTAT AGTGAGTCGTATTAATTTC | 126 |
| T7-crRNA-45-rs642803-C | CACAGACAGGCAATTCTATCTACAACAGTAGAAATTCCCT ATAGTGAGTCGTATTAATTTC | 127 |
| T7-crRNA-45-rs642803-T | CACAGACAGACAATTCTATCTACAACAGTAGAAATTCCCT ATAGTGAGTCGTATTAATTTC | 128 |
| T7-crRNA-gyrB | TCGCGCTTGTCGCGCAGACGAATGATCTACAACAGTAGAA ATTCCCTATAGTGAGTCGTATTAATTTC | 129 |

### Primers used for detection by DNA amplification through LAMP combined with Cas12a:

**Table 6 Primers used for amplifying gyrB-1**

| **Name** | **Sequence** | **SEQ ID No. :** |
|---|---|---|
| LAMP-gyrB-1-F3 | CATGGTGCGTTTCTGGCC | 130 |
| LAMP-gyrB-1-B3 | CGGCGTTTTGTTCTTGTTCA | 131 |
| LAMP-gyrB-1-FIP | ACAACTCACGCAGACGTTTCGCAACCTTCA CCAATGTGACCG | 132 |
| LAMP-gyrB-1-BIP | GTTCCTCAACTCCGGCGTTTCCGATGCCGC CTTCATAGTGG | 133 |
| LAMP-gyrB-1-LoopF | CAGAATTTCATATTCGAACT | 134 |
| LAMP-gyrB-1-LoopB | GACGGCAAAGAAGACCACTT | 135 |

**Table 7 Primers used for amplifying gyrB-2**

| **Name** | **Sequence** | **SEQ ID No. :** |
|---|---|---|
| LAMP-gyrB-2-F3 | CGACGGCAAAGAAGACCA | 136 |
| LAMP-gyrB-2-B3 | AGCCTGCCAGGTGAGTAC | 137 |
| LAMP-gyrB-2-FIP | CGGGTGGATCGGCGTTTTGTTCACTATGAA GGCGGCATCA | 138 |
| LAMP-gyrB-2-BIP | GTATTGGCGTCGAAGTGGCGTTCGCTGCGG AATGTTGTTG | 139 |
| LAMP-gyrB-2-LoopF | TTGTTCAGATATTCAACGAACG | 1.40 |
| LAMP-gyrB-2-LoopB | GTGGAACGATGGCTTCCAGG | 141 |

**Table 8 Primers used for amplifying a rs1467558 site**

| **Name** | **Sequence** | **SEQ ID No. :** |
|---|---|---|
| LAMP-rs1467558-F3 | CAGCTGTAGACCATAAGCC | 142 |
| LAMP-rs1467558-B3 | GTGGCTGAGCATCGTTAT | 143 |
| LAMP-rs1467558-FIP | ACTATGAAAGGAGTCCATATCTGAAGGAAT TCAGGTAGTGGTTTGGGA | 144 |
| LAMP-rs1467558-BIP | GCTTCAGCCTACTGCAAATCCTACGCGTTG TCATTGAAAG | 145 |
| LAMP-rs1467558-LoopF | TCAATATTAGTGTTATTGCTTG | 146 |
| LAMP-rs1467558-LoopB | TGGTGGAAGATTTGGACAGGAC | 147 |

**Table 9 Primers used for amplifying a rs5082 site**

| **Name** | **Sequence** | **SEQ ID No. :** |
|---|---|---|
| LAMP-rs5082-F3 | GCTGGAAAGGTCAAGGGAC | 148 |
| LAMP-rs5082-B3 | GGGGTTTGTTGCACAGTCC | 149 |
| LAMP-rs5082-FIP | CAAAGGTAGAAGCAAAGGCAGGAGGTTTGC CCAAGGTCACACAG | 150 |
| LAMP-rs5082-BIP | CTGGGAAGAGGGAGGGCTCAGTGTTGCCAC ACTTTCACTGG | 151 |
| LAMP-rs5082-LoopF | GTGAGCGGGTGGGGTGCT | 152 |
| LAMP-rs5082-LoopB | TCTAAGTCTTCCAGCACGGGATC | 153 |

**Table 10 Primers used for Detection by RPA Amplification combined with Cas12**

| **Name** | **Sequence** | **SEQ ID No. :** |
|---|---|---|
| RPA-gyrB-1-F | ATATGAAATTCTGGCGAAACGTCTGCGTGAGTTG | 154 |
| RPA-gyrB-2-F | AAACGTCTGCGTGAGTTGTCGTTCCTCAACTCC | 155 |
| RPA-gyrB-R | ACTTCGACGCCAATACCGTCTTTTTCAGTGGAG | 156 |

**Table 11 Primers used for determining Cas12b having a trans-cleavage activity:**

| **Oligo Name** | **Sequence (5'-3')** | **SEQ ID No. :** |
|---|---|---|
| pUC18-1-F | ATCTGAGAAGTGGCACTTATCGCAACTTTCTACTGAGGTC ATAGCTGTTTCCTGTGTGA | 157 |
| pUC18-1-R | GTCCTCTAGACCCCTATAGTGAGTCGTATTAATTTCAT GATTACGAATTCGAGCTCGGT | 158 |
| pUC18-2-F | CCACTTTCCAGGTGGCAAAGCCCGTTGAGCTTCTCAAA TCTGAGAAGTGGCACTTATC | 159 |
| pUC18-2-R | TGGAAAGTGGCCATTGGCACACCCGTTGAAAAATTCTG TCCTCTAGACCCCTATAGTGA | 160 |
| T7-crRNA-F | GAAATTAATACGACTCACTATAGGG | 161 |
| ZL-sgRNA-T1-R | TCAGTAGAAAGTTGCGATAAGTGC | 162 |
| ZLsgRNA-DNMT1-3-R | AACAGACATGGACCATCAGGGTG | 163 |
| target-T1-F | TTTCTGTTTGTTATCGCAACTTTCTACTGAATTCAAGC TTTACTCTAGAAAGAGGAGAAAGGATCC | 164 |
| target-T1-R | GGATCCTTTCTCCTCTTTCTAGAGTAAAGCTTGAATTC AGTAGAAAGTTGCGATAACAAACAGAAA | 165 |
| target-DNMT1-3-R-FAM-5' | GTCACGCCACTTGACAGGCGAGTAACAGACATGGACCA TCAGGAAACATT | 166 |
| target-T1-R | GGATCCTTTCTCCTCTTTCTAGAGTAAAGCTTGAATTC AGTAGAAAGTTGCGATAACAAACAGAAA | 167 |
| target-T1-F | TTTCTGTTTGTTATCGCAACTTTCTACTGAATTCAAGC TTTACTCTAGAAAGAGGAGAAAGGATCC | 168 |

**Table 12 Primers used for sensitivity test of trans reaction of Cas12b**

| **Oligo Name** | **Sequence (5'-3')** | **SEQ ID No. :** |
|---|---|---|
| sgRNA-DNMT1-3-F | CCTGATGGTCCATGTCTGTTGGTCATAGCTGTTTCCTGTG TG | 169 |
| sgRNA-DNM1-3-R | TGGACCATCAGGGTGCCACTTCTCAGATTTGAGAAG | 170 |
| T7-crRNA-F | GAAATTAATACGACTCACTATAGGG | 171 |
| ZLsgRNA-DNMT1-3-R | AACAGACATGGACCATCAGGGTG | 172 |
| DNMT1-3(TTC PAM)-F | AATGTTCCCTGATGGTCCATGTCTGTTACTCGCCTGTC AAGTGGCGTGAC | 173 |
| DNMT1-3(TTC PAM)-R | GTCACGCCACTTGACAGGCGAGTAACAGACATGGACCA TCAGGGAACATT | 174 |
| LAMP-DNM-F3 | gtgaacgttcccttagcact | 175 |
| LAMP-DNM-B3 | gggagggcagaactag tcc | 176 |
| LAMP-DNM-FIP | cgcccacttgacaggcgagtaactgccacttattgggtc agc | 177 |
| LAMP-DNM-BIP | gcgtgttccccagagtgacttagcagcttcctcctcct t | 178 |
| LAMP-DNM-LoopF | aggaaacattaacgtactgatg | 179 |
| LAMP-DNM-LoopB | ttccttttatttcccttcagc | 180 |
| DNMT1-3(TTC PAM)-R | GTCACGCCACTTGACAGGCGAGTAACAGACATGGACCA TCAGGGAACATT | 181 |
| DNMT1-3(TTC PAM)-F | AATGTTCCCTGATGGTCCATGTCTGTTACTCGCCTGTC AAGTGGCGTGAC | 182 |

**Table 13 Other Sequences Involved in the present invention**

| **Name** | **Sequence** | **SEQ ID No. :** |
|---|---|---|
| AacCas12b sgRNA sequence | GTCTAGAGGACAGAATTTTTCAACGGGTGTGCCAATGG CCACTTTCCAGGTGGCAAAGCCCGTTGAGCTTCTCAAA TCTGAGAAGTGGCACcctgatggtccatgtctgtt | 183 |
| A guide sequence targeting a target DNMT-1-3 | cctgatggtccatgtctgtt | 184 |
| Single-stranded target sequence | gtcacgccacttgacaggcgagtaacagacatggacca tcagggaacatt | 185 |
| Double-stranded target sequence: | gtcacgccacttgacaggcgagtaacagacatggacca tcagggaacatt | 186 |
| Amino acid sequence of the AacCas12b protein | MAVKSIKVKLRLDDMPEIRAGLWKLHKEVNAGVRYYTE WLSLLRQENLYRRSPNGDGEQECDKTAEECKAELLERL RARQVENGHRGPAGSDDELLQLARQLYELLVPQAIGAK GDAQQIARKFLSPLADKDAVGGLGIAKAGNKPRWVRMR EAGEPGWEEEKEKAETRKSADRTADVLRALADFGLKPL MRVYTDSEMSSVEWKPLRKGQAVRTWDRDMFQQAIERM MSWESWNQRVGQEYAKLVEQKNRFEQKNFVGQEHLVHL VNQLQQDMKEASPGLESKEQTAHYVTGRALRGSDKVFE KWGKLAPDAPFDLYDAEIKNVQRRNTRRFGSHDLFAKL AEPEYQALWREDASFLTRYAVYNSILRKLNHAKMFATF TLPDATAHPIWTRFDKLGGNLHQYTFLFNEFGERRHAI RFHKLLKVENGVAREVDDVTVPISMSEQLDNLLPRDPN EPIALYFRDYGAEQHFTGEFGGAKIQCRRDQLAHMHRR RGARDVYLNVSVRVQSQSEARGERRPPYAAVFRLVGDN HRAFVHFDKLSDYLAEHPDDGKLGSEGLLSGLRVMSVD LGLRTSASISVFRVARKDELKPNSKGRVPFFFPIKGND | 187 |

All documents mentioned in the present invention are hereby incorporated by reference as if each document is individually incorporated by reference. Furthermore, it should be understood that after reading the above teachings of the present invention, those skilled in the art can make various changes or modifications to the present invention, and these equivalent forms also fall within the scope defined by the appended claims of the present application.

## Claims

1. A method for detecting a target nucleic acid molecule, comprising adding a guide RNA, a Cas protein, a nucleic acid probe and a buffer solution into a system comprising the target nucleic acid molecule to be detected, and then detecting the nucleic acid probe.

2. The method for detecting a target nucleic acid molecule according to claim 1, wherein the Cas protein is Cas12a or a Cas protein having a collateral single-stranded DNA cleavage activity similar to that of the Cas12a;
the Cas12a is preferably one of FnCas12a, AsCas12a, LbCas12a, Lb5Cas12a, HkCas12a, OsCas12a, TsCas12a, BbCas12a, BoCas12a or Lb4Cas12a; and the Cas12a is preferably LbCas12a.

3. The method for detecting a target nucleic acid molecule according to claim 1, wherein the guide RNA refers to an RNA that guides the Cas protein to specifically bind to a target DNA.

4. The method for detecting a target nucleic acid molecule according to claim 1, wherein the nucleic acid probe is a single-stranded DNA; the single-stranded DNA is preferably a fluorescently-labeled single-stranded DNA; the single-stranded DNA is preferably a fluorescent probe that is labeled with a fluorescent group HEX at a 5' terminal and labeled with a quenching group BHQ1 at a 3' terminal; preferably,
the method for detecting the nucleic acid probe is preferably a fluorescence detection method; and the fluorescence detection method is preferably a detection method using a microplate reader or a fluorescence spectrophotometer.

5. The method for detecting a target nucleic acid molecule according to any one of claims 1 to 4, wherein the target nucleic acid molecule to be detected in the reaction system of the target nucleic acid molecule to be detected is obtained by amplification.

6. The method for detecting a target nucleic acid molecule according to claim 5, wherein the detection method can detect a pathogenic microorganism, gene mutation, or a specific target DNA.

7. The method according to claim 1, wherein the Cas protein comprises Cas12b (i.e., C2cl).

8. Use of a Cas protein in a method for detecting a target nucleic acid molecule.

9. The use according to claim 8, wherein when a target DNA, a guide RNA and a Cas protein form a ternary complex, the complex cleaves other single-stranded DNA molecules in the system; and preferably, the guide RNA refers to an RNA that guides the Cas protein to specifically bind to the target DNA.

10. A kit for detecting a target nucleic acid molecule, comprising a guide RNA, a Cas protein and a nucleic acid probe.

11. A detection system for detecting a target nucleic acid molecule, comprising:
(a) a Cas protein, which is Cas12a or a Cas protein having a collateral single-stranded DNA cleavage activity similar to that of the Cas12a;
(b) a guide RNA that guides the Cas protein to specifically bind to the target nucleic acid molecule; and
(c) a nucleic acid probe which is a single-stranded DNA;
wherein the target nucleic acid molecule is a target DNA.

12. The detection system according to claim 11, wherein the Cas protein having a collateral single-stranded DNA cleavage activity similar to that of Cas12a is selected from a group consisting of Cas12b (i.e., C2c1).

13. The detection system according to claim 11, wherein the nucleic acid probe comprises a single-stranded DNA carrying a detectable label.

14. A kit for detecting a target nucleic acid molecule, comprising:
i) a first container and a Cas protein in the first container, the Cas protein being Cas12a or Cas protein having a collateral single-stranded DNA cleavage activity similar to that of the Cas12a;
ii) an optional second container and a guide RNA in the second container, the guide RNA guiding the Cas protein to specifically bind to the target nucleic acid molecule;
iii) a third container and a nucleic acid probe in the third container;
iv) an optional fourth container and a buffer solution in the fourth container;
wherein the target nucleic acid molecule is a target DNA.

15. A method for detecting whether a target nucleic acid molecule exists in a sample, comprising the following steps:
(a) providing the detection system for detecting a target nucleic acid molecule according to claim 11, wherein the detection system further comprises a sample to be detected; and
(b) detecting whether the nucleic acid probe in the detection system is cleaved by a Cas protein, wherein the cleavage is a trans-cleavage of a collateral single-stranded DNA;
wherein if the nucleic acid probe is cleaved by the Cas protein, then it indicates the presence of the target nucleic acid molecule in the sample; and if the nucleic acid probe is not cleaved by the Cas protein, then it indicates the absence of the target nucleic acid molecule in the sample.

16. The method according to claim 15, wherein a method for amplifying the nucleic acid is selected from a group consisting of PCR amplification, LAMP amplification, RPA amplification, ligase chain reaction, branched DNA amplification, NASBA, SDA, transcription-mediated amplification, rolling circle amplification, HDA, SPIA, NEAR, TMA, and SMAP2.

17. The method according to claim 15, wherein when the upstream and downstream (in a range of -20 nt to +20 nt, preferably in a range of -15 nt to +15 nt, and more preferably in a range of -10 nt to +10 nt) of a target site lack a PAM sequence, nucleic acid amplification is carried out using a PAM-introducing primer.

18. The method according to claim 17, wherein the PAM-introducing primer has a structure of formula I in 5'-3':
P1-P2-P3 (I)
wherein,
P1 is a 5' segment sequence that is located at the 5' terminal and is complementary or non-complementary to the sequence of the target nucleic acid molecule;
P2 is a PAM sequence; and
P3 is a 3' segment sequence that is located at the 3' terminal and is complementary to the sequence of the target nucleic acid molecule.

19. The method according to claim 15, wherein when the upstream and downstream (in a range of -20 nt to +20 nt, preferably in a range of -15 nt to +15 nt, and more preferably in a range of -10 nt to +10 nt) of the target site contain the PAM sequence, then a primer containing or not containing the PAM sequence can be used, and the amplified amplification product contains the PAM sequence.

20. Use of a Cas protein in preparation of a detection reagent or a kit for detecting a target nucleic acid molecule based on collateral single-stranded DNA cleavage, wherein the Cas protein is Cas12a or a Cas protein having a collateral single-stranded DNA cleavage activity similar to that of the Cas 12a.

21. The use according to claim 20, wherein the Cas protein having a collateral single-stranded DNA cleavage activity similar to that of the Cas12a is selected from a group consisting of Cas12b (or C2c1).


Sequence Listing

<110>  SHANGHAI TOLO BIOTECHNOLOGY COMPANY LIMITED
       SHANGHAI INSTITUTES FOR BIOLOGICAL SCIENCES, CHINESE ACADEMY OF
SCIENCES

<120>  USE OF CAS PROTEIN, METHOD FOR DETECTING TARGET NUCLEIC ACID
MOLECULE, AND KIT

<130>  P2018-0447

<150>  CN201710573752.0
<151>  2017-07-14

<160>  187

<170>  PatentIn version 3.5

<210>  1
<211>  50
<212>  DNA
<213>  Artificial sequence

<400>  1
aatgtttcct gatggtccat gtctgttact cgcctgtcaa gtggcgtgac                50


<210>  2
<211>  50
<212>  DNA
<213>  Artificial sequence

<400>  2
gtcacgccac ttgacaggcg agtaacagac atggaccatc aggaaacatt                50


<210>  3
<211>  50
<212>  DNA
<213>  Artificial sequence

<400>  3
gtcacgccac ttgacaggcg agtaacagac atggaccatc aggaaacatt                50


<210>  4
<211>  50
<212>  DNA
<213>  Artificial sequence

<400>  4
gtcacgccac ttgacaggcg agtaacagac atggaccatc aggaaacatt                50


<210>  5
<211>  66
<212>  DNA
<213>  Artificial sequence

<400>  5
tttctgtttg ttatcgcaac tttctactga attcaagctt tactctagaa agaggagaaa      60

ggatcc                                                                66

<210>  6
<211>  66
<212>  DNA
<213>  Artificial sequence

<400>  6
ggatcctttc tcctctttct agagtaaagc ttgaattcag tagaaagttg cgataacaaa      60

cagaaa                                                                  66


<210>  7
<211>  66
<212>  DNA
<213>  Artificial sequence

<400>  7
tttctgtttg ttatcgcaac tttctactga attcaagctt tactctagaa agaggagaaa      60

ggatcc                                                                  66


<210>  8
<211>  66
<212>  DNA
<213>  Artificial sequence

<400>  8
ggatcctttc tcctctttct agagtaaagc ttgaattcag tagaaagttg cgataacaaa      60

cagaaa                                                                  66


<210>  9
<211>  66
<212>  DNA
<213>  Artificial sequence

<400>  9
tttctgtttg ttatcgcaac tttctactga attcaagctt tactctagaa agaggagaaa      60

ggatcc                                                                  66


<210>  10
<211>  66
<212>  DNA
<213>  Artificial sequence

<400>  10
ggatcctttc tcctctttct agagtaaagc ttgaattcag tagaaagttg cgataacaaa      60

cagaaa                                                                  66


<210>  11
<211>  50
<212>  DNA
<213>  Artificial sequence

<400>  11

gtcacgccac ttgacaggcg agtaacagac atggaccatc aggtttcatt 50

<210> 12
<211> 50
<212> DNA
<213> Artificial sequence

<400> 12
gtcacgccac ttgacaggcg agtaacagac atggaccatc aggccccatt 50

<210> 13
<211> 50
<212> DNA
<213> Artificial sequence

<400> 13
gtcacgccac ttgacaggcg agtaacagac atggaccatc agggggcatt 50

<210> 14
<211> 50
<212> DNA
<213> Artificial sequence

<400> 14
aatgaaacct gatggtccat gtctgttact cgcctgtcaa gtggcgtgac 50

<210> 15
<211> 50
<212> DNA
<213> Artificial sequence

<400> 15
aatggggcct gatggtccat gtctgttact cgcctgtcaa gtggcgtgac 50

<210> 16
<211> 50
<212> DNA
<213> Artificial sequence

<400> 16
   aatgccccct gatggtccat gtctgttact cgcctgtcaa gtggcgtgac 50

<210> 17
<211> 11
<212> DNA
<213> Artificial sequence

<400> 17
acaaacagaa a 11

<210> 18
<211> 16
<212> DNA
<213> Artificial sequence

<400> 18

cgataacaaa cagaaa 16

<210> 19
<211> 22
<212> DNA
<213> Artificial sequence

<400> 19
aagttgcgat aacaaacaga aa 22

<210> 20
<211> 28
<212> DNA
<213> Artificial sequence

<400> 20
agtagaaagt tgcgataaca aacagaaa 28

<210> 21
<211> 34
<212> DNA
<213> Artificial sequence

<400> 21
gaattcagta gaaagttgcg ataacaaaca gaaa 34

<210> 22
<211> 24
<212> DNA
<213> Artificial sequence

<400> 22
gaattcagta gaaagttgcg ataa 24

<210> 23
<211> 18
<212> DNA
<213> Artificial sequence

<400> 23
agtagaaagt tgcgataa 18

<210> 24
<211> 12
<212> DNA
<213> Artificial sequence

<400> 24
aagttgcgat aa 12

<210> 25
<211> 12
<212> DNA
<213> Artificial sequence

<220>
<221>  misc_feature
<222>  (7)..(12)
<223>  n is a, c, g, or t

<400>  25
cgataa                                                                  12


<210>  26
<211>  25
<212>  DNA
<213>  Artificial sequence


<220>
<221>  misc_feature
<222>  (1)..(25)
<223>  n is a, c, g, or t

<400>  26
nnnnnnnnnn nnnnnnnnnn nnnnn                                             25


<210>  27
<211>  25
<212>  DNA
<213>  Artificial sequence


<220>
<221>  misc_feature
<222>  (1)..(25)
<223>  n is a, c, g, or t

<400>  27
nnnnnnnnnn nnnnnnnnnn nnnnn                                             25


<210>  28
<211>  66
<212>  DNA
<213>  Artificial sequence

<400>  28
ggatcctttc tcctctttct agagtaaagc ttgaattcag tagaaagttg cgataacaaa      60

cagaaa                                                                  66


<210>  29
<211>  28
<212>  DNA
<213>  Artificial sequence

<400>  29
cacaattcca cacaacatac gagccgga                                          28


<210>  30
<211>  27
<212>  DNA
<213>  Artificial sequence

<400>  30
tgtagccgta gttaggccac cacttca                                          27


<210>  31
<211>  31
<212>  DNA
<213>  Artificial sequence

<400>  31
agttttgtta tcgcaacttt ctactgaatt c                                     31


<210>  32
<211>  31
<212>  DNA
<213>  Artificial sequence

<400>  32
agttttgata tcgcaacttt ctactgaatt c                                     31


<210>  33
<211>  31
<212>  DNA
<213>  Artificial sequence

<400>  33
agttttgtaa tcgcaacttt ctactgaatt c                                     31


<210>  34
<211>  31
<212>  DNA
<213>  Artificial sequence

<400>  34
agttttgttt tcgcaacttt ctactgaatt c                                     31


<210>  35
<211>  31
<212>  DNA
<213>  Artificial sequence

<400>  35
agttttgtta acgcaacttt ctactgaatt c                                     31


<210>  36
<211>  31
<212>  DNA
<213>  Artificial sequence

<400>  36
agttttgtta tggcaacttt ctactgaatt c                                     31


<210>  37
<211>  31
<212>  DNA
<213>  Artificial sequence

<400>  37
agttttgtta tcccaacttt ctactgaatt c                                    31


<210>  38
<211>  31
<212>  DNA
<213>  Artificial sequence

<400>  38
agttttgtta tcggaacttt ctactgaatt c                                    31


<210>  39
<211>  31
<212>  DNA
<213>  Artificial sequence

<400>  39
agttttgtta tcgctacttt ctactgaatt c                                    31


<210>  40
<211>  31
<212>  DNA
<213>  Artificial sequence

<400>  40
agttttgtta tcgcatcttt ctactgaatt c                                    31


<210>  41
<211>  31
<212>  DNA
<213>  Artificial sequence

<400>  41
agttttgtta tcgcaagttt ctactgaatt c                                    31


<210>  42
<211>  29
<212>  DNA
<213>  Artificial sequence

<400>  42
aaaagttatc gcaactttct actgaattc                                       29


<210>  43
<211>  39
<212>  DNA
<213>  Artificial sequence

<400>  43
agttttgtta tcgcaacatt ctactgaatt cggtcatag                            39


<210>  44
<211>  39
<212>  DNA
<213>  Artificial sequence

<400>  44
agttttgtta tcgcaactat ctactgaatt cggtcatag                                39


<210>  45
<211>  39
<212>  DNA
<213>  Artificial sequence

<400>  45
agttttgtta tcgcaactta ctactgaatt cggtcatag                                39


<210>  46
<211>  39
<212>  DNA
<213>  Artificial sequence

<400>  46
agttttgtta tcgcaacttt gtactgaatt cggtcatag                                39


<210>  47
<211>  39
<212>  DNA
<213>  Artificial sequence

<400>  47
agttttgtta tcgcaacttt caactgaatt cggtcatag                                39


<210>  48
<211>  39
<212>  DNA
<213>  Artificial sequence

<400>  48
agttttgtta tcgcaacttt cttctgaatt cggtcatag                                39


<210>  49
<211>  39
<212>  DNA
<213>  Artificial sequence

<400>  49
agttttgtta tcgcaacttt ctagtgaatt cggtcatag                                39


<210>  50
<211>  39
<212>  DNA
<213>  Artificial sequence

<400>  50
agttttgtta tcgcaacttt ctacagaatt cggtcatag                                39


<210>  51
<211>  31
<212>  DNA
<213>  Artificial sequence

<400>  51
agtttagtta tcgcaacttt ctactgaatt c                                    31


<210>  52
<211>  31
<212>  DNA
<213>  Artificial sequence

<400>  52
agttatgtta tcgcaacttt ctactgaatt c                                    31


<210>  53
<211>  31
<212>  DNA
<213>  Artificial sequence

<400>  53
agtattgtta tcgcaacttt ctactgaatt c                                    31


<210>  54
<211>  28
<212>  DNA
<213>  Artificial sequence

<400>  54
agttgtcgtt cctcaactcc ggcgtttc                                       28


<210>  55
<211>  28
<212>  DNA
<213>  Artificial sequence

<400>  55
tcgacgccaa taccgtcttt ttcagtgg                                       28


<210>  56
<211>  26
<212>  DNA
<213>  Artificial sequence

<400>  56
ctgcctttgc ttctaccttt gcctgt                                         26


<210>  57
<211>  25
<212>  DNA
<213>  Artificial sequence

<400>  57
ttgcttctac ctttgcctgt tctgg                                          25


<210>  58
<211>  25
<212>  DNA
<213>  Artificial sequence

<400>  58
ttttctggct ggggatggcc gatgg                                              25


<210>  59
<211>  50
<212>  DNA
<213>  Artificial sequence

<400>  59
agcaataaca ctaatattga ttccttcaga tatggactcc tttcatagta                   50


<210>  60
<211>  39
<212>  DNA
<213>  Artificial sequence

<400>  60
ttgattcctt cagatatgga ctcctttcat agtataacg                               39


<210>  61
<211>  37
<212>  DNA
<213>  Artificial sequence

<400>  61
tgagcatcgt tattcttacg cgttgtcatt gaaagag                                 37


<210>  62
<211>  25
<212>  DNA
<213>  Artificial sequence

<400>  62
agcctgggca acgagtgaaa ctctg                                              25


<210>  63
<211>  27
<212>  DNA
<213>  Artificial sequence

<400>  63
acaggaggga caaaggccta agtgtcc                                            27


<210>  64
<211>  38
<212>  DNA
<213>  Artificial sequence

<400>  64
catcatagga ttgggaaaag gacatttcag tcattcag                                38


<210>  65
<211>  48
<212>  DNA
<213>  Artificial sequence

<400>  65
agagtccttc tttctcaatt tttcagaata atttagtact ttgggtac                    48


<210>  66
<211>  40
<212>  DNA
<213>  Artificial sequence

<400>  66
cagtactgaa aaaacctgcc tatcaataaa agccctagac                             40


<210>  67
<211>  24
<212>  DNA
<213>  Artificial sequence

<400>  67
gcttcaccgg ctacctttgc tcct                                              24


<210>  68
<211>  21
<212>  DNA
<213>  Artificial sequence

<400>  68
ttcacctgca ggccccgcag g                                                 21


<210>  69
<211>  39
<212>  DNA
<213>  Artificial sequence

<400>  69
cctgactttc aactctgtct ccttcctctt tttacagta                              39


<210>  70
<211>  28
<212>  DNA
<213>  Artificial sequence

<400>  70
tgctgtgact gcttgtagat ggccatgg                                          28


<210>  71
<211>  36
<212>  DNA
<213>  Artificial sequence

<400>  71
agtttccaga cctcagtgca caagatactt ttctac                                 36


<210>  72
<211>  36
<212>  DNA
<213>  Artificial sequence

<400>  72
acctcagtgc acaagatact tttctacgtc atccac                                36


<210>  73
<211>  33
<212>  DNA
<213>  Artificial sequence

<400>  73
agctccagtg gatggaagat ctttgagatc cag                                   33


<210>  74
<211>  40
<212>  DNA
<213>  Artificial sequence

<400>  74
agtcaaagag attcatgcct gggactttaa tcacatttat                            40


<210>  75
<211>  33
<212>  DNA
<213>  Artificial sequence

<400>  75
atgcctggga ctttaatcac atttatcgga agg                                   33


<210>  76
<211>  31
<212>  DNA
<213>  Artificial sequence

<400>  76
caaatctgtc tccacctctc agctcacctt g                                     31


<210>  77
<211>  34
<212>  DNA
<213>  Artificial sequence

<400>  77
ttcttgaacc caaactcacc tggcatttaa actg                                  34


<210>  78
<211>  33
<212>  DNA
<213>  Artificial sequence

<400>  78
aaactcacct ggcatttaaa ctgactctgt aag                                   33


<210>  79
<211>  33
<212>  DNA
<213>  Artificial sequence

<400>  79
aaactcacct ggcatttaaa ctgtctctgt aag                                    33


<210>  80
<211>  27
<212>  DNA
<213>  Artificial sequence

<400>  80
tgccgaggct gagttcagct actctcc                                           27


<210>  81
<211>  26
<212>  DNA
<213>  Artificial sequence

<400>  81
acacagcacc gtgggtcaga ccttgc                                            26


<210>  82
<211>  26
<212>  DNA
<213>  Artificial sequence

<400>  82
tgggtcagac tttgccggtg agagtc                                            26


<210>  83
<211>  26
<212>  DNA
<213>  Artificial sequence

<400>  83
tgggtcagac tttgctggtg agagtc                                            26


<210>  84
<211>  27
<212>  DNA
<213>  Artificial sequence

<400>  84
agcagtggcc atgtgatgct gatgatg                                           27


<210>  85
<211>  27
<212>  DNA
<213>  Artificial sequence

<400>  85
ccccggctct gttggctttg agaattg                                           27


<210>  86
<211>  33
<212>  DNA
<213>  Artificial sequence

<400>  86
ctctgttggc tttgagaatt gcctgtctgt gtc                                    33


<210>  87
<211>  33
<212>  DNA
<213>  Artificial sequence

<400>  87
ctctgttggc tttgagaatt gtctgtctgt gtc                                    33


<210>  88
<211>  25
<212>  DNA
<213>  Artificial sequence

<400>  88
accgatacct ggcagccctt ggatg                                             25


<210>  89
<211>  12
<212>  DNA
<213>  Artificial sequence


<220>
<221>  misc_feature
<222>  (1)..(12)
<223>  n is a, c, g, or t

<400>  89
nnnnnnnnnn nn                                                           12


<210>  90
<211>  25
<212>  DNA
<213>  Artificial sequence

<400>  90
gaaattaata cgactcacta taggg                                             25


<210>  91
<211>  68
<212>  DNA
<213>  Artificial sequence

<400>  91
gaattcagta gaaagttgcg ataaatctac aacagtagaa attccctata gtgagtcgta       60

ttaatttc                                                                68


<210>  92
<211>  59
<212>  DNA
<213>  Artificial sequence

<400>  92
agaaagttgc gataaatcta caacagtaga aattccctat agtgagtcgt attaatttc        59


<210>  93
<211>  60
<212>  DNA
<213>  Artificial sequence

<400>  93
tagaaagttg cgataaatct acaacagtag aaattcccta tagtgagtcg tattaatttc       60


<210>  94
<211>  61
<212>  DNA
<213>  Artificial sequence

<400>  94
gtagaaagtt gcgataaatc tacaacagta gaaattccct atagtgagtc gtattaattt       60

c                                                                       61


<210>  95
<211>  62
<212>  DNA
<213>  Artificial sequence

<400>  95
agtagaaagt tgcgataaat ctacaacagt agaaattccc tatagtgagt cgtattaatt       60

tc                                                                      62


<210>  96
<211>  67
<212>  DNA
<213>  Artificial sequence

<400>  96
gagtaacaga catggaccat cagatctaca acagtagaaa ttccctatag tgagtcgtat       60

taatttc                                                                 67


<210>  97
<211>  67
<212>  DNA
<213>  Artificial sequence

<400>  97
gacatggacc atcaggaaac attatctaca acagtagaaa ttccctatag tgagtcgtat       60

taatttc                                                                 67


<210>  98
<211>  67
<212>  DNA
<213>  Artificial sequence

<400>  98

aggcgagtaa cagacatgga ccaatctaca acagtagaaa ttccctatag tgagtcgtat 60

taatttc 67

<210> 99
<211> 67
<212> DNA
<213> Artificial sequence

<400> 99
tgacaggcga gtaacagaca tggatctaca acagtagaaa ttccctatag tgagtcgtat 60

taatttc 67

<210> 100
<211> 60
<212> DNA
<213> Artificial sequence

<400> 100
agacatggac catcagatct acaacagtag aaattcccta tagtgagtcg tattaatttc 60

<210> 101
<211> 62
<212> DNA
<213> Artificial sequence

<400> 101
acagacatgg accatcagat ctacaacagt agaaattccc tatagtgagt cgtattaatt 60

tc 62

<210> 102
<211> 64
<212> DNA
<213> Artificial sequence

<400> 102
taacagacat ggaccatcag atctacaaca gtagaaattc cctatagtga gtcgtattaa 60

tttc 64

<210> 103
<211> 48
<212> DNA
<213> Artificial sequence

<400> 103
gacatggacc atcaggaaac attccctata gtgagtcgta ttaatttc 48

<210> 104
<211> 48
<212> DNA
<213> Artificial sequence

<400> 104
aggcgagtaa cagacatgga ccaccctata gtgagtcgta ttaatttc 48

<210>  105
<211>  48
<212>  DNA
<213>  Artificial sequence

<400>  105
tgacaggcga gtaacagaca tggccctata gtgagtcgta ttaatttc                48


<210>  106
<211>  61
<212>  DNA
<213>  Artificial sequence

<400>  106
cctcttccca gaacaggatc tacaacagta gaaattccct atagtgagtc gtattaattt      60

c                                                                   61


<210>  107
<211>  61
<212>  DNA
<213>  Artificial sequence

<400>  107
cctcttccca gcacaggatc tacaacagta gaaattccct atagtgagtc gtattaattt      60

c                                                                   61


<210>  108
<211>  61
<212>  DNA
<213>  Artificial sequence

<400>  108
ctgaagcgtt atactatatc tacaacagta gaaattccct atagtgagtc gtattaattt      60

c                                                                   61


<210>  109
<211>  61
<212>  DNA
<213>  Artificial sequence

<400>  109
ctgaagcgtt gtactatatc tacaacagta gaaattccct atagtgagtc gtattaattt      60

c                                                                   61


<210>  110
<211>  60
<212>  DNA
<213>  Artificial sequence

<400>  110
ttttatctga atgattatct acaacagtag aaattcccta tagtgagtcg tattaatttc      60

<210>  111
<211>  60
<212>  DNA
<213>  Artificial sequence

<400>  111
ttttatctga atgactatct acaacagtag aaattcccta tagtgagtcg tattaatttc        60


<210>  112
<211>  61
<212>  DNA
<213>  Artificial sequence

<400>  112
aaaaaagagt gagtaccatc tacaacagta gaaattccct atagtgagtc gtattaattt        60

c                                                                         61


<210>  113
<211>  61
<212>  DNA
<213>  Artificial sequence

<400>  113
aaaaaagagt gggtaccatc tacaacagta gaaattccct atagtgagtc gtattaattt        60

c                                                                         61


<210>  114
<211>  61
<212>  DNA
<213>  Artificial sequence

<400>  114
ggtagaaggt ccaggagatc tacaacagta gaaattccct atagtgagtc gtattaattt        60

c                                                                         61


<210>  115
<211>  61
<212>  DNA
<213>  Artificial sequence

<400>  115
ggtagaaggt ctaggagatc tacaacagta gaaattccct atagtgagtc gtattaattt        60

c                                                                         61


<210>  116
<211>  60
<212>  DNA
<213>  Artificial sequence

<400>  116
gggcagggga gtactgatct acaacagtag aaattcccta tagtgagtcg tattaatttc        60

<210>  117
<211>  60
<212>  DNA
<213>  Artificial sequence

<400>  117
gggcagggga ctactgatct acaacagtag aaattcccta tagtgagtcg tattaatttc        60


<210>  118
<211>  59
<212>  DNA
<213>  Artificial sequence

<400>  118
tcagtggatg atgtaatcta caacagtaga aattccctat agtgagtcgt attaatttc        59


<210>  119
<211>  59
<212>  DNA
<213>  Artificial sequence

<400>  119
tcagtggatg acgtaatcta caacagtaga aattccctat agtgagtcgt attaatttc        59


<210>  120
<211>  61
<212>  DNA
<213>  Artificial sequence

<400>  120
ggaaattctc cttccgaatc tacaacagta gaaattccct atagtgagtc gtattaattt        60

c                                                                         61


<210>  121
<211>  61
<212>  DNA
<213>  Artificial sequence

<400>  121
ggaaattctc cttccaaatc tacaacagta gaaattccct atagtgagtc gtattaattt        60

c                                                                         61


<210>  122
<211>  60
<212>  DNA
<213>  Artificial sequence

<400>  122
tcttacagag tcagttatct acaacagtag aaattcccta tagtgagtcg tattaatttc        60


<210>  123
<211>  60
<212>  DNA
<213>  Artificial sequence

<400>  123
tcttacagag ccagttatct acaacagtag aaattcccta tagtgagtcg tattaatttc        60


<210>  124
<211>  61
<212>  DNA
<213>  Artificial sequence

<400>  124
gtcttacaga gacagttatc tacaacagta gaaattccct atagtgagtc gtattaattt        60

c                                                                         61


<210>  125
<211>  59
<212>  DNA
<213>  Artificial sequence

<400>  125
ctggactctc accggatcta caacagtaga aattccctat agtgagtcgt attaatttc         59


<210>  126
<211>  59
<212>  DNA
<213>  Artificial sequence

<400>  126
ctggactctc accagatcta caacagtaga aattccctat agtgagtcgt attaatttc         59


<210>  127
<211>  61
<212>  DNA
<213>  Artificial sequence

<400>  127
cacagacagg caattctatc tacaacagta gaaattccct atagtgagtc gtattaattt        60

c                                                                         61


<210>  128
<211>  61
<212>  DNA
<213>  Artificial sequence

<400>  128
cacagacaga caattctatc tacaacagta gaaattccct atagtgagtc gtattaattt        60

c                                                                         61


<210>  129
<211>  68
<212>  DNA
<213>  Artificial sequence

<400>  129
tcgcgcttgt cgcgcagacg aatgatctac aacagtagaa attccctata gtgagtcgta        60

ttaatttc                                                              68


<210>  130
<211>  18
<212>  DNA
<213>  Artificial sequence

<400>  130
catggtgcgt ttctggcc                                                    18


<210>  131
<211>  20
<212>  DNA
<213>  Artificial sequence

<400>  131
cggcgttttg ttcttgttca                                                  20


<210>  132
<211>  42
<212>  DNA
<213>  Artificial sequence

<400>  132
acaactcacg cagacgtttc gcaaccttca ccaatgtgac cg                         42


<210>  133
<211>  41
<212>  DNA
<213>  Artificial sequence

<400>  133
gttcctcaac tccggcgttt ccgatgccgc cttcatagtg g                          41


<210>  134
<211>  20
<212>  DNA
<213>  Artificial sequence

<400>  134
cagaatttca tattcgaact                                                  20


<210>  135
<211>  20
<212>  DNA
<213>  Artificial sequence

<400>  135
gacggcaaag aagaccactt                                                  20


<210>  136
<211>  18
<212>  DNA
<213>  Artificial sequence

<400>  136

cgacggcaaa gaagacca                                                    18

<210>  137
<211>  18
<212>  DNA
<213>  Artificial sequence

<400>  137
agcctgccag gtgagtac                                                    18

<210>  138
<211>  40
<212>  DNA
<213>  Artificial sequence

<400>  138
cgggtggatc ggcgttttgt tcactatgaa ggcggcatca                            40

<210>  139
<211>  40
<212>  DNA
<213>  Artificial sequence

<400>  139
gtattggcgt cgaagtggcg ttcgctgcgg aatgttgttg                            40

<210>  140
<211>  22
<212>  DNA
<213>  Artificial sequence

<400>  140
ttgttcagat attcaacgaa cg                                               22

<210>  141
<211>  20
<212>  DNA
<213>  Artificial sequence

<400>  141
gtggaacgat ggcttccagg                                                  20

<210>  142
<211>  19
<212>  DNA
<213>  Artificial sequence

<400>  142
cagctgtaga ccataagcc                                                   19

<210>  143
<211>  18
<212>  DNA
<213>  Artificial sequence

<400>  143

gtggctgagc atcgttat                                                    18

<210>  144
<211>  48
<212>  DNA
<213>  Artificial sequence

<400>  144
actatgaaag gagtccatat ctgaaggaat tcaggtagtg gtttggga                  48

<210>  145
<211>  40
<212>  DNA
<213>  Artificial sequence

<400>  145
gcttcagcct actgcaaatc ctacgcgttg tcattgaaag                            40

<210>  146
<211>  22
<212>  DNA
<213>  Artificial sequence

<400>  146
tcaatattag tgttattgct tg                                              22

<210>  147
<211>  22
<212>  DNA
<213>  Artificial sequence

<400>  147
tggtggaaga tttggacagg ac                                              22

<210>  148
<211>  19
<212>  DNA
<213>  Artificial sequence

<400>  148
gctggaaagg tcaagggac                                                  19

<210>  149
<211>  19
<212>  DNA
<213>  Artificial sequence

<400>  149
ggggtttgtt gcacagtcc                                                  19

<210>  150
<211>  44
<212>  DNA
<213>  Artificial sequence

<400>  150

caaaggtaga agcaaaggca ggaggtttgc ccaaggtcac acag 44

<210> 151
<211> 41
<212> DNA
<213> Artificial sequence

<400> 151
ctgggaagag ggagggctca gtgttgccac actttcactg g 41

<210> 152
<211> 18
<212> DNA
<213> Artificial sequence

<400> 152
gtgagcgggt ggggtgct 18

<210> 153
<211> 23
<212> DNA
<213> Artificial sequence

<400> 153
tctaagtctt ccagcacggg atc 23

<210> 154
<211> 34
<212> DNA
<213> Artificial sequence

<400> 154
atatgaaatt ctggcgaaac gtctgcgtga gttg 34

<210> 155
<211> 33
<212> DNA
<213> Artificial sequence

<400> 155
aaacgtctgc gtgagttgtc gttcctcaac tcc 33

<210> 156
<211> 33
<212> DNA
<213> Artificial sequence

<400> 156
acttcgacgc caataccgtc tttttcagtg gag 33

<210> 157
<211> 59
<212> DNA
<213> Artificial sequence

<400> 157

atctgagaag tggcacttat cgcaactttc tactgaggtc atagctgttt cctgtgtga 59

<210> 158
<211> 59
<212> DNA
<213> Artificial sequence

<400> 158
gtcctctaga cccctatagt gagtcgtatt aatttcatga ttacgaattc gagctcggt 59

<210> 159
<211> 58
<212> DNA
<213> Artificial sequence

<400> 159
ccactttcca ggtggcaaag cccgttgagc ttctcaaatc tgagaagtgg cacttatc 58

<210> 160
<211> 59
<212> DNA
<213> Artificial sequence

<400> 160
tggaaagtgg ccattggcac acccgttgaa aaattctgtc ctctagaccc ctatagtga 59

<210> 161
<211> 25
<212> DNA
<213> Artificial sequence

<400> 161
gaaattaata cgactcacta taggg 25

<210> 162
<211> 24
<212> DNA
<213> Artificial sequence

<400> 162
tcagtagaaa gttgcgataa gtgc 24

<210> 163
<211> 23
<212> DNA
<213> Artificial sequence

<400> 163
aacagacatg gaccatcagg gtg 23

<210> 164
<211> 66
<212> DNA
<213> Artificial sequence

<400> 164

tttctgtttg ttatcgcaac tttctactga attcaagctt tactctagaa agaggagaaa 60

ggatcc 66

<210> 165
<211> 66
<212> DNA
<213> Artificial sequence

<400> 165
ggatcctttc tcctctttct agagtaaagc ttgaattcag tagaaagttg cgataacaaa 60

cagaaa 66

<210> 166
<211> 50
<212> DNA
<213> Artificial sequence

<400> 166
gtcacgccac ttgacaggcg agtaacagac atggaccatc aggaaacatt 50

<210> 167
<211> 66
<212> DNA
<213> Artificial sequence

<400> 167
ggatcctttc tcctctttct agagtaaagc ttgaattcag tagaaagttg cgataacaaa 60

cagaaa 66

<210> 168
<211> 66
<212> DNA
<213> Artificial sequence

<400> 168
tttctgtttg ttatcgcaac tttctactga attcaagctt tactctagaa agaggagaaa 60

ggatcc 66

<210> 169
<211> 42
<212> DNA
<213> Artificial sequence

<400> 169
cctgatggtc catgtctgtt ggtcatagct gtttcctgtg tg 42

<210> 170
<211> 36
<212> DNA
<213> Artificial sequence

<400> 170
tggaccatca gggtgccact tctcagattt gagaag 36

<210>  171
<211>  25
<212>  DNA
<213>  Artificial sequence

<400>  171
gaaattaata cgactcacta taggg                                          25


<210>  172
<211>  23
<212>  DNA
<213>  Artificial sequence

<400>  172
aacagacatg gaccatcagg gtg                                            23


<210>  173
<211>  50
<212>  DNA
<213>  Artificial sequence

<400>  173
aatgttccct gatggtccat gtctgttact cgcctgtcaa gtggcgtgac               50


<210>  174
<211>  50
<212>  DNA
<213>  Artificial sequence

<400>  174
gtcacgccac ttgacaggcg agtaacagac atggaccatc agggaacatt               50


<210>  175
<211>  20
<212>  DNA
<213>  Artificial sequence

<400>  175
gtgaacgttc ccttagcact                                                20


<210>  176
<211>  19
<212>  DNA
<213>  Artificial sequence

<400>  176
gggagggcag aactagtcc                                                 19


<210>  177
<211>  41
<212>  DNA
<213>  Artificial sequence

<400>  177
cgccacttga caggcgagta actgccactt attgggtcag c                        41

<210>  178
<211>  39
<212>  DNA
<213>  Artificial sequence

<400>  178
gcgtgttccc cagagtgact tagcagcttc ctcctcctt                              39


<210>  179
<211>  22
<212>  DNA
<213>  Artificial sequence

<400>  179
aggaaacatt aacgtactga tg                                                22


<210>  180
<211>  21
<212>  DNA
<213>  Artificial sequence

<400>  180
ttccttttat ttcccttcag c                                                 21


<210>  181
<211>  50
<212>  DNA
<213>  Artificial sequence

<400>  181
gtcacgccac ttgacaggcg agtaacagac atggaccatc agggaacatt                  50


<210>  182
<211>  50
<212>  DNA
<213>  Artificial sequence

<400>  182
aatgttccct gatggtccat gtctgttact cgcctgtcaa gtggcgtgac                  50


<210>  183
<211>  111
<212>  DNA
<213>  Artificial sequence

<400>  183
gtctagagga cagaattttt caacgggtgt gccaatggcc actttccagg tggcaaagcc      60

cgttgagctt ctcaaatctg agaagtggca ccctgatggt ccatgtctgt t               111


<210>  184
<211>  20
<212>  DNA
<213>  Artificial sequence

<400>  184
cctgatggtc catgtctgtt                                                   20


<210>  185
<211>  50
<212>  DNA
<213>  Artificial sequence

<400>  185
gtcacgccac ttgacaggcg agtaacagac atggaccatc agggaacatt                  50


<210>  186
<211>  50
<212>  DNA
<213>  Artificial sequence

<400>  186
gtcacgccac ttgacaggcg agtaacagac atggaccatc agggaacatt                  50


<210>  187
<211>  1129
<212>  PRT
<213>  Alicyclobacillus acidoterrestris

<400>  187

Met Ala Val Lys Ser Ile Lys Val Lys Leu Arg Leu Asp Asp Met Pro
1               5                   10                  15

Glu Ile Arg Ala Gly Leu Trp Lys Leu His Lys Glu Val Asn Ala Gly
            20                  25                  30

Val Arg Tyr Tyr Thr Glu Trp Leu Ser Leu Leu Arg Gln Glu Asn Leu
        35                  40                  45

Tyr Arg Arg Ser Pro Asn Gly Asp Gly Glu Gln Glu Cys Asp Lys Thr
    50                  55                  60

Ala Glu Glu Cys Lys Ala Glu Leu Leu Glu Arg Leu Arg Ala Arg Gln
65                  70                  75                  80

Val Glu Asn Gly His Arg Gly Pro Ala Gly Ser Asp Asp Glu Leu Leu
                85                  90                  95

Gln Leu Ala Arg Gln Leu Tyr Glu Leu Leu Val Pro Gln Ala Ile Gly
            100                 105                 110

Ala Lys Gly Asp Ala Gln Gln Ile Ala Arg Lys Phe Leu Ser Pro Leu
        115                 120                 125

Ala Asp Lys Asp Ala Val Gly Gly Leu Gly Ile Ala Lys Ala Gly Asn
    130                 135                 140

Lys Pro Arg Trp Val Arg Met Arg Glu Ala Gly Glu Pro Gly Trp Glu
145                 150                 155                 160

Glu Glu Lys Glu Lys Ala Glu Thr Arg Lys Ser Ala Asp Arg Thr Ala
                165                 170                 175

Asp Val Leu Arg Ala Leu Ala Asp Phe Gly Leu Lys Pro Leu Met Arg
            180                 185                 190

Val Tyr Thr Asp Ser Glu Met Ser Ser Val Glu Trp Lys Pro Leu Arg
        195                 200                 205

Lys Gly Gln Ala Val Arg Thr Trp Asp Arg Asp Met Phe Gln Gln Ala
    210                 215                 220

Ile Glu Arg Met Met Ser Trp Glu Ser Trp Asn Gln Arg Val Gly Gln
225                 230                 235                 240

Glu Tyr Ala Lys Leu Val Glu Gln Lys Asn Arg Phe Glu Gln Lys Asn
                245                 250                 255

Phe Val Gly Gln Glu His Leu Val His Leu Val Asn Gln Leu Gln Gln
            260                 265                 270

Asp Met Lys Glu Ala Ser Pro Gly Leu Glu Ser Lys Glu Gln Thr Ala
        275                 280                 285

His Tyr Val Thr Gly Arg Ala Leu Arg Gly Ser Asp Lys Val Phe Glu
    290                 295                 300

Lys Trp Gly Lys Leu Ala Pro Asp Ala Pro Phe Asp Leu Tyr Asp Ala
305                 310                 315                 320

Glu Ile Lys Asn Val Gln Arg Arg Asn Thr Arg Arg Phe Gly Ser His
                325                 330                 335

Asp Leu Phe Ala Lys Leu Ala Glu Pro Glu Tyr Gln Ala Leu Trp Arg
            340                 345                 350

Glu Asp Ala Ser Phe Leu Thr Arg Tyr Ala Val Tyr Asn Ser Ile Leu
        355                 360                 365

Arg Lys Leu Asn His Ala Lys Met Phe Ala Thr Phe Thr Leu Pro Asp
    370                 375                 380

Ala Thr Ala His Pro Ile Trp Thr Arg Phe Asp Lys Leu Gly Gly Asn

385                 390                 395                 400

Leu His Gln Tyr Thr Phe Leu Phe Asn Glu Phe Gly Glu Arg Arg His
                405                 410                 415

Ala Ile Arg Phe His Lys Leu Leu Lys Val Glu Asn Gly Val Ala Arg
            420                 425                 430

Glu Val Asp Asp Val Thr Val Pro Ile Ser Met Ser Glu Gln Leu Asp
        435                 440                 445

Asn Leu Leu Pro Arg Asp Pro Asn Glu Pro Ile Ala Leu Tyr Phe Arg
    450                 455                 460

Asp Tyr Gly Ala Glu Gln His Phe Thr Gly Glu Phe Gly Gly Ala Lys
465                 470                 475                 480

Ile Gln Cys Arg Arg Asp Gln Leu Ala His Met His Arg Arg Arg Gly
                485                 490                 495

Ala Arg Asp Val Tyr Leu Asn Val Ser Val Arg Val Gln Ser Gln Ser
            500                 505                 510

Glu Ala Arg Gly Glu Arg Arg Pro Pro Tyr Ala Ala Val Phe Arg Leu
        515                 520                 525

Val Gly Asp Asn His Arg Ala Phe Val His Phe Asp Lys Leu Ser Asp
    530                 535                 540

Tyr Leu Ala Glu His Pro Asp Asp Gly Lys Leu Gly Ser Glu Gly Leu
545                 550                 555                 560

Leu Ser Gly Leu Arg Val Met Ser Val Asp Leu Gly Leu Arg Thr Ser
                565                 570                 575

Ala Ser Ile Ser Val Phe Arg Val Ala Arg Lys Asp Glu Leu Lys Pro
            580                 585                 590

Asn Ser Lys Gly Arg Val Pro Phe Phe Phe Pro Ile Lys Gly Asn Asp
        595                 600                 605

Asn Leu Val Ala Val His Glu Arg Ser Gln Leu Leu Lys Leu Pro Gly
    610                 615                 620

Glu Thr Glu Ser Lys Asp Leu Arg Ala Ile Arg Glu Glu Arg Gln Arg
625                 630                 635                 640

Thr Leu Arg Gln Leu Arg Thr Gln Leu Ala Tyr Leu Arg Leu Leu Val
            645                 650                 655

Arg Cys Gly Ser Glu Asp Val Gly Arg Arg Glu Arg Ser Trp Ala Lys
        660                 665                 670

Leu Ile Glu Gln Pro Val Asp Ala Ala Asn His Met Thr Pro Asp Trp
    675                 680                 685

Arg Glu Ala Phe Glu Asn Glu Leu Gln Lys Leu Lys Ser Leu His Gly
    690                 695                 700

Ile Cys Ser Asp Lys Glu Trp Met Asp Ala Val Tyr Glu Ser Val Arg
705                 710                 715                 720

Arg Val Trp Arg His Met Gly Lys Gln Val Arg Asp Trp Arg Lys Asp
            725                 730                 735

Val Arg Ser Gly Glu Arg Pro Lys Ile Arg Gly Tyr Ala Lys Asp Val
        740                 745                 750

Val Gly Gly Asn Ser Ile Glu Gln Ile Glu Tyr Leu Glu Arg Gln Tyr
    755                 760                 765

Lys Phe Leu Lys Ser Trp Ser Phe Phe Gly Lys Val Ser Gly Gln Val
    770                 775                 780

Ile Arg Ala Glu Lys Gly Ser Arg Phe Ala Ile Thr Leu Arg Glu His
785                 790                 795                 800

Ile Asp His Ala Lys Glu Asp Arg Leu Lys Lys Leu Ala Asp Arg Ile
            805                 810                 815

Ile Met Glu Ala Leu Gly Tyr Val Tyr Ala Leu Asp Glu Arg Gly Lys
        820                 825                 830

Gly Lys Trp Val Ala Lys Tyr Pro Pro Cys Gln Leu Ile Leu Leu Glu
    835                 840                 845

Glu Leu Ser Glu Tyr Gln Phe Asn Asn Asp Arg Pro Pro Ser Glu Asn
    850                 855                 860

Asn Gln Leu Met Gln Trp Ser His Arg Gly Val Phe Gln Glu Leu Ile
865                 870                 875                 880

Asn Gln Ala Gln Val His Asp Leu Leu Val Gly Thr Met Tyr Ala Ala
            885                 890                 895

Phe Ser Ser Arg Phe Asp Ala Arg Thr Gly Ala Pro Gly Ile Arg Cys
            900                 905                 910

Arg Arg Val Pro Ala Arg Cys Thr Gln Glu His Asn Pro Glu Pro Phe
        915                 920                 925

Pro Trp Trp Leu Asn Lys Phe Val Val Glu His Thr Leu Asp Ala Cys
    930                 935                 940

Pro Leu Arg Ala Asp Asp Leu Ile Pro Thr Gly Glu Gly Glu Ile Phe
945                 950                 955                 960

Val Ser Pro Phe Ser Ala Glu Glu Gly Asp Phe His Gln Ile His Ala
                965                 970                 975

Asp Leu Asn Ala Ala Gln Asn Leu Gln Gln Arg Leu Trp Ser Asp Phe
            980                 985                 990

Asp Ile Ser Gln Ile Arg Leu Arg Cys Asp Trp Gly Glu Val Asp Gly
        995                 1000                1005

Glu Leu Val Leu Ile Pro Arg Leu Thr Gly Lys Arg Thr Ala Asp
    1010                1015                1020

Ser Tyr Ser Asn Lys Val Phe Tyr Thr Asn Thr Gly Val Thr Tyr
    1025                1030                1035

Tyr Glu Arg Glu Arg Gly Lys Lys Arg Arg Lys Val Phe Ala Gln
    1040                1045                1050

Glu Lys Leu Ser Glu Glu Glu Ala Glu Leu Leu Val Glu Ala Asp
    1055                1060                1065

Glu Ala Arg Glu Lys Ser Val Val Leu Met Arg Asp Pro Ser Gly
    1070                1075                1080

Ile Ile Asn Arg Gly Asn Trp Thr Arg Gln Lys Glu Phe Trp Ser
    1085                1090                1095

Met Val Asn Gln Arg Ile Glu Gly Tyr Leu Val Lys Gln Ile Arg
    1100                1105                1110

Ser Arg Val Pro Leu Gln Asp Ser Ala Cys Glu Asn Thr Gly Asp
    1115                1120                1125

Ile